(19) Europäisches Patentamt
European Patent Office
Office européen des brevets

(11) EP 1 854 451 B1

(12) **EUROPEAN PATENT SPECIFICATION**

(45) Date of publication and mention of the grant of the patent:
**27.04.2011 Bulletin 2011/17**

(51) Int Cl.:
***A61K 8/90*** *(2006.01)* ***A61K 8/92*** *(2006.01)*
***A61Q 1/06*** *(2006.01)*

(21) Application number: **07008772.1**

(22) Date of filing: **30.04.2007**

(54) **Cosmetic compositions containing block copolymers and corresponding long-wearing cosmetic product system**

Kosmetische Zusammensetzungen mit Blockcopolymeren und entsprechendes System von dauerhaften Kosmetikprodukten

Compositions cosmétiques contenant des copolymères séquencés et produits correspondants à tenue prolongée

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **03.05.2006 US 417986**
**03.05.2006 US 417975**
**03.05.2006 US 417981**
**03.05.2006 US 417977**
**03.05.2006 US 418327**
**30.10.2006 US 589396**
**30.10.2006 US 589696**

(43) Date of publication of application:
**14.11.2007 Bulletin 2007/46**

(73) Proprietor: **L'Oréal**
**75008 Paris (FR)**

(72) Inventors:
- **Bui, Hy Si**
**Piscataway, NJ 08854 (US)**
- **Lu, Shaoxiang**
**Kang Qiao Town, Shanghai 201315 (CN)**
- **Pradier, François**
**New York, NY 10128 (US)**
- **Mercado, Ramon**
**Clifton, NJ 07011 (US)**
- **Blin, Xavier**
**75015 Paris (FR)**
- **Arnaud, Pascal**
**94240 L'Hay les Roses (FR)**
- **McDermott, Padraig**
**92190 Meudon (FR)**

(74) Representative: **Le Coupanec, Pascale A.M.P.**
**Nony & Associés**
**3, rue de Penthièvre**
**75008 Paris (FR)**

(56) References cited:
**EP-A- 1 582 203**
**US-A- 6 083 516**
**US-A1- 2003 068 344**
**US-A1- 2005 228 115**

EP 1 854 451 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

There have been many developments in connection with improving comfort, wear, shine and/or longevity of cosmetic compositions for the face, eye, lips nails or hair. Commercially available lip treatment compositions such as lip glosses and lipsticks possess a certain level of gloss or shine depending of their composition. Efforts have been made through the use of high refractive index fluids to further enhance the shine or gloss of such products, but the wear of gloss or shine is limited. Moreover, these lip treatment compositions are tacky and uncomfortable to apply due, oftentimes, to the presence of high molecular weight polymers having a high viscosity which are used to maintain the wear of shine/gloss.

While other efforts to improve shine/gloss involve the use of silicone fluids, wear of shine leaves much to be desired.

Therefore, one of the aspects of the present invention is to provide a lip treatment composition which is comfortable to apply and wear, and has long lasting shine/gloss.

Accordingly, a first aspect of the present invention is directed to a cosmetic composition comprising :

(a) at least one block copolymer having at least one hard segment and at least one soft segment;
(b) at least one tackifier component;
(c) at least one solvent capable of solubilizing the at least one hard segment; and
(d) optionally, at least one colorant, and

wherein the at least one hard segment has a $T_g$ value of 50 °C or more, and the at least one soft segment has a $T_g$ value of 20 °C or less,
wherein (a) is a mixture of: (i) at least one di-block thermoplastic elastomer of an A-B type copolymer and (ii) at least one tri-block thermoplastic elastomer of an A-B-A type copolymer, wherein A corresponds to styrene and B corresponds to rubber,
wherein the at least one tackifier is a hydrogenated styrene/methyl styrene/indene copolymer, and wherein the at least one solvent capable of solubilizing the at least one hard segment has a viscosity equal or less than 200 cps at room temperature and has a weight average molecular weight equal or less than 500, with said solvent (c) being nonvolatile solvent, chosen among monoesters, diesters, triesters; mixed aliphatic and/or aromatic, polar oils; natural or synthetic esters of formula $R_1COOR_2$, wherein $R_1$ is a higher fatty residue comprising 7 to 19 carbon atoms, and $R_2$ is a branched hydrocarbon-based chain comprising 3 to 20 carbon atoms; synthetic esters of formula $R^3COR^4$, wherein $R^3$ is a $C_3$ to $C_{19}$ alkyl radical, and $R^4$ is a $C_3$ to $C_{20}$ alkyl radical; fatty alcohols comprising at least 12 carbon atoms; cyclic hydrocarbons; aromatic hydrocarbons; primary, secondary or tertiary amines and mixtures thereof.

Thus, it has been surprisingly discovered that a lip treatment composition containing: (a) a block copolymer having at least one hard segment and at least one soft segment, (b) a tackifier, and (c) at least a solvent capable of solubilizing the at least one hard segment, in mixture or not with at least one solvent capable of solubilizing the soft segment of the block copolymer, can deliver a comfortable, long lasting shine or gloss when applied onto lips.

The physical and rheological properties of block copolymers can be controlled by using specific types of solvents capable of solubilizing the hard and/or soft block segments. A solvent capable of solubilizing the soft segment causes the block copolymer to possess a morphology and rheology different from that obtained using a solvent capable of solubilizing the hard segment. Similarly, the physical properties of a block copolymer solution based on a mixture of solvents capable of solubilizing both hard and soft segments are different from those obtained when using solvents capable of solubilizing only the soft segments or solvents capable of solubilizing only the hard segments.

Furthermore, the present invention is directed according to another of its aspects to a long-wearing cosmetic product system and method of making-up a keratinous substrate using said cosmetic product system.
Compositions used to enhance cosmetic products are known in the art. Such compositions, sometimes referred to as "topcoats", include those that are applied over top of basecoat compositions, such as a lipstick, in order to impart attributes such as gloss, shine and lubricity which are not typically afforded by basecoat compositions. These enhancement products utilize a variety of polymeric fluids in order to impart gloss, shine and lubricity.

While such topcoat compositions may provide these types of enhancements, it has been found that they are not particularly transfer resistant. As a result, these topcoats must be re-applied throughout the course of a day in order to maintain gloss, shine and lubricity on the basecoat cosmetic composition.

Thus, the present invention is directed to a cosmetic method of making-up a keratinous substrate involving :

(a) providing a keratinous substrate;
(b) applying a basecoat composition onto the keratinous substrate, the basecoat composition containing:

(i) at least one polyorganosiloxane containing polymer chosen among

(1) the (co)polymers comprising at least one organosiloxane unit and at least two other groups capable of forming hydrogen interactions chosen from an ester group, a sulfonamide group, a carbamate group, a thiocarbamate group, a urea group, a thio-urea group, an oxamido group, a guanidino group, a biguanidino group, an amide group, and mixtures thereof;
(2) the silicone polyamide copolymers and their mixtures

(ii) at least one silicone film former;
(iii) at least one volatile oil; and
(iv) at least one colorant; and

(c) applying a topcoat composition over top of the basecoat composition, the topcoat composition being the composition as defined previously.

Other than in the operating examples, or where otherwise indicated, all numbers expressing quantities of ingredients or reaction conditions are to be understood as being modified in all instances by the term "about".

COSMETIC COMPOSITION

BLOCK COPOLYMER

The block copolymers of the present invention are characterized by the presence of at least one "hard" segment, and at least one "soft" segment. Aside from their compositional nature, the hard and soft segments of the block copolymers of the present invention are defined in terms of their respective glass transition temperatures, "$T_g$". More particularly, the hard segment has a $T_g$ of 50°C or more, whereas the soft segment has a $T_g$ of 20°C or less. The glass transition temperature $T_g$ for the hard block can range from 50°C to 150°C; 60°C to 125°C; 70°C to 120°C; 80°C to 110°C. The glass transition temperature $T_g$ for the soft segment of the block copolymer can range from 20°C to -150°C; 0°C to -135°C; -10°C to -125°C; -25°C to -100°C. A more in depth explanation can be found in U.S. Patents 5,294,438 and 6,403,070, the entire contents of which are hereby incorporated by reference.

The block copolymer which is employed by the present invention is a thermoplastic elastomer. The hard segments of the thermoplastic elastomer comprise styrene.

The soft segments of the thermoplastic elastomer comprise ethylene/butylene copolymers.

The block copolymer for use in the present invention is a combination of di-block and tri-block copolymers of styrene-ethylene/butylene-styrene, commercially available from Shell Chemical Company under trade name Kraton G1657M.

The block copolymer is generally present in the cosmetic composition in an amount ranging from greater than 0% to 50% by weight; greater than 0% to 40% by weight; greater than 0% to 30% by weight; greater than 0% to 20% by weight; greater than 0% to 10% by weight, based on the weight of the composition.

TACKIFIERS

A substance is described as a tackifier if, by adding it to a block copolymer, the resulting composition has the properties of a pressure sensitive adhesive. In general, tackifiers can be divided into four different families in terms of their chemistry : hydrocarbon resins, terpenes, amorphous (i.e. non-crystalline) rosins, rosin esters and their derivatives, and pure monomer resins. These tackifiers are characterized by their compatibility with at least one segment of the block copolymer. By the term "compatible", it is meant that when the block copolymer and tackifier are mixed, the combination of at least one segment of the block copolymer with the tackifier forms a polymer blend having a single glass transition temperature $T_g$ which may be measured by DMA, DSC or neutron light scattering.

The compatibility of the block copolymer and the tackifier may also be defined in terms of solubility parameters. The solubility parameter δ according to the Hansen solubility space is defined in the article "Solubility Parameter Values" by Eric A. Grulke in the work "Polymer Handbook" 3rd edition, Chapter VII, pages 519-559, the entire content of which is hereby incorporated by reference, by the relationship:

$$\delta = (d_D^2 + d_P^2 + d_H^2)^{1/2} ,$$

in which:

**[0022]** - $d_D$ characterizes the London dispersion forces resulting from the formation of dipoles induced during molecular impacts,

**[0023]** - $d_P$ characterizes the forces of Debye interactions between permanent dipoles,

**[0024]** - $d_H$ characterizes the forces of specific interactions (hydrogen bond, acid/base or donor/acceptor type and the like). The definition of the solvents in the three-dimensional solubility space according to Hansen is given in the article

by C. M. Hansen: "The three-dimensional solubility parameters" J. Paint Technol., 39, 105(1967), the entire content of which is hereby incorporated by reference.

The at least one tackifier used in the present invention will have a solubility parameter corresponding to a number δ and the block copolymer will have at least one segment whose solubility parameter corresponds to δ ± 2, preferably δ ± 1.7, more preferably δ ± 1.5, more preferably δ ± 1.3, more preferably δ ± 1.0, more preferably δ ± 0.7, more preferably δ ± 0.5, and more preferably δ ± 0.3.

In some embodiments, the tackifier may have a softening point (Ring and Ball, as measured by ASTM E-28) of 80°C to 150°C, preferably 100°C to 130°C. In other embodiments the tackifier may be liquid and have an R and B softening point of between about -70°C and 70°C.

The tackifiers according to the present invention are hydrogenated hydrocarbon resins such as a hydrogenated styrene/methyl styrene/indene copolymer e.g., styrene/methyl styrene/indene copolymers which include R1090, R1100, R7100, S1100, and S5100, all which are commercially available from Eastman Chemical under the trade name Regalite®. In other embodiments, aliphatic or aromatic hydrocarbon-based tackifying resins, for instance the resins sold under the name "Piccotac" and "Hercotac" from Hercules or "Escorez" from Exxon, may also be used. It is also to be understood that mixtures of tackifiers may also be employed without departing from the spirit of the invention.

A particularly preferred tackifier for use in the present invention is a hydrogenated hydrocarbon resin such, for example, a hydrogenated styrene/methyl styrene/indene copolymer, commercially available from Eastman under the tradename Regalite® R1100.

The tackifier is present in the present composition in an amount ranging from greater than 0% to 90% by weight; greater than 0% to 70% by weight; greater than 0% to 60% by weight; greater than 0% to 50% by weight; greater than 0% to 40%; greater than 0% to 30% by weight; greater than 0% to 20% by weight, based on the weight of the composition.

SOLVENTS

Solvents capable of solubilizing the hard segment of the block copolymer which may be used herein are typically characterized in terms of their viscosity at room temperature, average molecular weight and solubility parameter in relation to the at least one hard segment of the block copolymer.

The solvent capable of solubilizing the hard segment of the block copolymer will have a viscosity, at room temperature, of from 0,001 to 0,2 Pa·s; preferably from 0,001 to 0,15 Pa·s, more preferably from 0,001 to 0,1 Pa·s, more preferably from 0,002 to 0,6 Pa·s, and more preferably from 0,002 to 0,4 Pa·s.

The solvent capable of solubilizing the hard segment of the block copolymer used in the present invention will have a solubility parameter corresponding to a number δ' and the block copolymer will have at least one hard segment whose solubility parameter corresponds to δ' ± 2, preferably δ' ± 1.7, more preferably δ' ± 1.5, more preferably δ' ± 1.3, more preferably δ' ± 1.0, more preferably δ' ± 0.7, more preferably δ' ± 0.5, and more preferably δ' ± 0.3.

Nonvolatile solvents capable of solubilizing the hard segment of the block copolymer which are used in the invention are monoesters, diesters, triesters, mixed aliphatic and/or aromatic, polar oils such as: hydrocarbon-based oils of animal origin, such as perhydrosqualene; hydrocarbon-based plant oils such as liquid triglycerides of fatty acids and of glycerol, in which the fatty acids may have varied chain lengths, these chains being linear or branched, and saturated or unsaturated; these oils can be chosen, for example, from wheat germ oil, sunflower oil, corn oil, soybean oil, marrow oil, grapeseed oil, blackcurrant seed oil, sesame oil, hazelnut oil, apricot oil, macadamia oil, castor oil, avocado oil, karite butter, sweet almond oil, cotton oil, alfalfa oil, poppy oil, pumpkin oil, evening primrose oil, millet oil, barley oil, quinoa oil, olive oil, rye oil, safflower oil, candlenut oil, passion flower oil, musk rose oil and caprylic/capric acid triglycerides such as those sold by the company Stéarineries Dubois or those sold under the names Miglyol 810, 812 and 818 by the company Dynamit Nobel; natural or synthetic esters of formula $R_1COOR_2$, wherein $R_1$ is a higher fatty acid residue comprising 7 to 19 carbon atoms, and $R_2$ is a branched hydrocarbon-based chain comprising 3 to 20 carbon atoms, such as, for example, purcellin oil (cetostearyl octanoate), isopropyl myristate and alkyl or polyalkyl octanoates, decanoates or ricinoleates; synthetic ethers of formula $R^3COR^4$, wherein $R^3$ is a $C_3$ to $C_{19}$ alkyl radical, and $R^4$ is a $C_3$ to $C_{20}$ alkyl radical; fatty alcohols comprising at least 12 carbon atoms, such as octyldodecanol or oleyl alcohol; cyclic hydrocarbons such as (alkyl)cycloalkanes, wherein the alkyl chain is linear or branched, saturated or unsaturated and comprises 1 to 30 carbon atoms, such as cyclohexane or dioctylcyclohexane; aromatic hydrocarbons, for example, alkenes such as benzene, toluene, 2, 4-dimethyl-3-cyclohexene, dipentene, p-cymene, naphthalene or anthracene, and esters such as isostearyl benzoate; primary, secondary or tertiary amines such as triethanolamine; and mixtures thereof. In one embodiment, synthetic esters such as isopropyl myristate are used.

Preferred esters are those having a weight average molecular weight (Mw) in the range of 100 to 600, preferably from 100 to 500. Examples thereof include, but are not limited to, $C_{12-15}$ alkyl benzoate, isopropyl myristate (Mw=270), isopropyl palmitate (Mw=300), isononyl isononanoate, cetyl ethylhexanoate (Mw=368), neopentyl glycol diethylhexanoate (Mw=356), diisopropyl sebacate (Mw=286).

The solvent capable of solubilizing the hard segment of the block copolymer may typically be present in the composition of the invention in an amount of up to 85% by weight; greater than 0% to 75% by weight; greater than 0% to 55% by weight; greater than 0% to 45% by weight; greater than 0% to 40% by weight; greater than 0% to 30% by

weight; greater than 0% to 20% by weight; greater than 0% to 10% by weight; greater than 0% to 5% by weight, based on the weight of the composition.

According to a preferred embodiment of the present invention, this solvent may be mixed with at least one solvent capable of solubilizing the soft segment of the block copolymer.

Solvents capable of solubilizing the soft segment of the block copolymer which may be used herein are typically characterized in terms of their viscosity at room temperature, average molecular weight and solubility parameter in relation to the at least one soft segment of the block copolymer.

The solvent capable of solubilizing the soft segment of the block copolymer will have a viscosity, at room temperature, of from 0,001 to 0,5 Pa.s, preferably from 0,001 to 0,4 Pa.s, more preferably from 0,001 to 0,3 Pa.s , more preferably from 0,002 to 0,2 Pa.s, and more preferably from 0,002 to 0,1 Pa.s.

The solvent capable of solubilizing the soft segment of the block copolymer used in the present invention will have a solubility parameter corresponding to a number δ' and the block copolymer will have at least one soft segment whose solubility parameter corresponds to δ' ± 2, preferably δ' ± 1.7, more preferably δ' ± 1.5, more preferably δ' ± 1.3, more preferably δ' ± 1.0, more preferably δ' ± 0.7, more preferably δ' ± 0.5, and more preferably δ' ± 0.3.

The solvent capable of solubilizing the soft segment of the block copolymer may be selected from volatile solvents and nonvolatile solvents. The expression "volatile solvent" means a solvent that is capable of evaporating at room temperature from a support onto which it has been applied, in other words a solvent which has a measurable vapor pressure at room temperature. See, U.S. Patent 6,656,458, the entire content of which is hereby incorporated by reference.

Representative examples of suitable volatile organic solvents include, but are not limited to, volatile hydrocarbon-based oils. The expression "hydrocarbon-based oil" means oil containing only hydrogen and carbon atoms. Examples of volatile hydrocarbon-based oils include isoparaffins, i.e., branched alkanes containing from 8 to 16 carbon atoms, and in particular isododecane (also known as 2,2,4,4,6-pentamethylheptane). It is also possible to use mixtures of such isoparaffins. Other volatile hydrocarbon-based oils, such as petroleum distillates, can also be used.

Suitable nonvolatile solvents which can be used are those having an average molecular weight in the range of 150 to 450, preferably from 200 to 350. Examples thereof include, but are not limited to, hydrogenated polydecene, hydrogenated polyisobutene, isoeicosane, polydecene and polybutene.

The solvent capable of solubilizing the soft segment of the block copolymer may typically be present in the composition of the invention in an amount of up to 85% by weight; greater than 0% to 75% by weight; greater than 0% to 55% by weight; greater than 0% to 45% by weight; greater than 0% to 40% by weight; greater than 0% to 30% by weight; greater than 0% to 20% by weight; greater than 0% to 10% by weight; greater than 0% to 5% by weight, based on the weight of the composition.

According to a preferred embodiment of the present invention, at least one co-solvent having a high molecular weight and high viscosity may also be used in order to improve the flow and leveling of the lip treatment composition during application onto the lips, as well as its feel and comfort thereon.

Examples of suitable high viscosity co-solvents which are compatible with the hard segment of the block copolymer include, but are not limited to, capric/caprylic triglyceride (Mw=500), diisopropyl dimer dilinoleate (Mw=644), diisostearyl fumarate (Mw=620), diisostearyl malate (Mw=640), pentaerythrityl tetraoleate, neopentyl glycol diethylhexanoate, diethylhexyl sebacate and tricaprylate/tricaprate. The average molecular weight of these co-solvents is preferably from 500 to 1000, and more preferably from 500 to 800.

Examples of suitable high viscosity co-solvents which are compatible with the soft segment of the block copolymer include, but are not limited to, polyisobutene, hydrogenated polyisobutene, polybutene, hydrogenated polybutene, polydecene and hydrogenated polydecene. The average molecular weight of these co-solvents is preferably from 2,500 to 100, 000, and more preferably from 3,000 to 10,000.

These co-solvents may be employed in the composition of the invention in an amount of up to 50% by weight; greater than 0% to 40% by weight; greater than 0% to 30% by weight; greater than 0% to 25% by weight; all weights based on the weight of the composition.

According to yet another embodiment of the present invention, it has been found that the use of at least one homopolymer of the same type as that of the at least one solvent capable of solubilizing the soft segment, but having a average molecular weight of greater than 2000, improves the adhesion, thereby limiting the migration, of the lip treatment composition on the skin.

Examples of suitable homopolymers include, but are not limited to, polyisobutene, hydrogenated polyisobutene, polybutene, hydrogenated polybutene, polydecene and hydrogenated polydecene. The average molecular weight of these homopolymers is preferably from 2,500 to 100,000, and more preferably from 3,000 to 10,000.

The homopolymer can be present in the composition of the invention in an amount of from greater than 0% to 30% by weight; greater than 0% to 25% by weight; greater than 0% to 20% by weight; greater than 0% to 18% by weight; greater than 0% to 15% by weight, all weights based on the weight of the composition.

In the event that at least one solvent capable of solubilizing the hard segment, and at least one solvent capable

of solubilizing the soft segment are used in combination with one or more of the at least one co-solvent compatible with the hard segment, at least one co-solvent compatible with the soft segment, and at least one homopolymer, the mixture will have a viscosity of from 0,02 to 5 Pa.s, preferably from 0,02 to 2 Pa.s, and more preferably from 0,02 to 1,500 Pa.s. The viscosity of the mixture is determined using the formula:

$$\eta_{mix} = \prod_{i}^{n} \eta_i^{\phi_i}$$

wherein $\eta_{mix}$ represents the viscosity of the mixture, $\eta_i$ represents the viscosity of the individual components, and $\phi_i$ represents the weight fraction of the individual components.

COLORANT

The composition of the present invention may also contain at least one cosmetically acceptable colorant such as a pigment or dyestuff. Examples of suitable pigments include, but are not limited to, inorganic pigments, organic pigments, lakes, pearlescent pigments, irridescent or optically variable pigments, and mixtures thereof. A pigment should be understood to mean inorganic or organic, white or colored particles. Said pigments may optionally be surface-treated within the scope of the present invention but are not limited to treatments such as silicones, perfluorinated compounds, lecithin, and amino acids.

Representative examples of inorganic pigments useful in the present invention include those selected from the group consisting of rutile or anatase titanium dioxide, coded in the Color Index under the reference CI 77,891; black, yellow, red and brown iron oxides, coded under references CI 77,499, 77, 492 and, 77,491; manganese violet (CI 77,742); ultramarine blue (CI 77,007); chromium oxide (CI 77,288); chromium hydrate (CI 77,289); and ferric blue (CI 77,510) and mixtures thereof.

Representative examples of organic pigments and lakes useful in the present invention include, but are not limited to, D&C Red No. 19 (CI 45,170), D&C Red No. 9 (CI 15,585), D&C Red No. 21 (CI 45,380), D&C Orange No. 4 (CI 15,510), D&C Orange No. 5 (CI 45,370), D&C Red No. 27 (CI 45,410), D&C Red No. 13 (CI 15,630), D&C Red No. 7 (CI 15,850), D&C Red No. 6 (CI 15,850), D&C Yellow No. 5 (CI 19,140), D&C Red No. 36 (CI 12,085), D&C Orange No. 10 (CI 45,425), D&C Yellow No. 6 (CI 15,985), D&C Red No. 30 (CI 73,360), D&C Red No.3 (CI 45,430) and the dye or lakes based on Cochineal Carmine (CI 75,570) and mixtures thereof.

Representative examples of pearlescent pigments useful in the present invention include those selected from the group consisting of the white pearlescent pigments such as mica coated with titanium oxide, mica coated with titanium dioxide, bismuth oxychloride, titanium oxychloride, colored pearlescent pigments such as titanium mica with iron oxides, titanium mica with ferric blue, chromium oxide and the like, titanium mica with an organic pigment of the above-mentioned type as well as those based on bismuth oxychloride and mixtures thereof.

The precise amount and type of colorant employed in the compositions of the present invention will depend on the color, intensity and use of the cosmetic composition and, as a result, will be determined by those skilled in the art of cosmetic formulation.

SHINE ENHANCING AGENTS

It may, at times, be desirable to provide cosmetic compositions having enhanced shine/gloss properties. In those instances, at least one shine enhancing agent would be employed in the composition.

Suitable shine enhancing agents include those compounds having a refractive index ranging from 1.45 to 1.60, and an average molecular weight of less than 15,000, preferably less than 10,000, preferably less than 2,000. Examples thereof include, but are not limited to, phenylated silicones such as those commercialized under the trade name "Abil AV 8853" by Goldschmidt, those commercialized under the trade names "DC 554", "DC 555", "DC 556", "SF 558" by Dow Corning, and those commercialized under the trade name "Silbione 70633 V 30" by Rhône-Poulenc.

Additional examples of suitable phenylated silicones include, but are not limited to, those commercialized by Wacker Silicones such as Belsil PDM 20, a phenylated silicone with a viscosity at 25° C of approximately 20 cSt ; Belsil PDM 200, a phenylated silicone with a viscosity at 25° C of approximately 200 cSt; Belsil PDM 1000, a phenylated silicone with a viscosity at 25° C of approximately 1000 cSt.

Additional examples of suitable shine enhancing agents include, but are not limited to, polycyclopentadiene, poly(propylene glycol) dibenzoate ($n_D$=1.5345), aminopropyl phenyl trimethicone ($n_D$=1.49-1.51), pentaerythrityl tetraoleate commercially available as Puresyn 4E68 ($n_D$=1.473) from ExxonMobil, and PPG-3 benzyl ether myristate commercially available as Crodamol STS ($n_D$=1.4696) from Croda Inc.

Particularly preferred shine enhancing agents are the phenylated silicones such as phenyl trimethicone, and trimethyl pentaphenyl trisiloxane, and esters such as pentaerythrityl tetraoleate, and PPG-3 benzyl ether myristate.

The shine enhancing agent may be present in the composition of the invention in an amount of up to 40% by weight; up to 30% by weight; up to 20% by weight; from 1 to 20% by weight; from 2 to 20% by weight, based on the weight of the composition.

MODIFIED SILICONES

The cosmetic compositions of the present invention may contain at least one modified silicone to improve the texture and comfort. Examples of suitable modified silicones include, but are not limited to, polyethyleneoxy- and/or polypropyleneoxy-modified silicone, alkoxy-modified silicone, hydroxyalkyl-modified silicone, acyloxyalkyl-modified silicone, alkyl-modified silicone, amino-modified silicone, epoxy-modified silicone, carboxyl-modified silicone, chloroalkyl-modified silicone, alkyl-higher-alcohol-ester-modified silicone, alcohol- modified silicone, polyether-modified silicone, phenyl-modified silicone, alkylpolyglyceryl-modified silicone, perfluoroalkyl polyether-co-modified silicone and fluorine-modified silicone.

The modified silicone may be present in the composition of the invention in an amount of up to 30% by weight; up to 25% by weight; up to 20% by weight; up to 10% by weight; up to 8% by weight, based on the weight of the composition.

WAXES

In some embodiments, it may be desirable to formulate cosmetic compositions in accordance with the present invention which are free of wax. However, in the event that a wax is employed, it will be present in an amount of from about 0.1% to about 30% by weight, based on the total weight of the composition. Suitable waxes are those generally used in cosmetics and dermatology. Examples thereof include, but are not limited to, those of natural origin such as beeswax, carnauba wax, candelilla wax, ouricury wax, Japan wax, cork fiber wax, sugar cane wax, paraffin wax, lignite wax, microcrystalline waxes, lanolin wax, montan wax, ozokerites and hydrogenated oils such as hydrogenated jojoba oil. Examples of suitable synthetic waxes include, but are not limited to, polyethylene waxes derived from the polymerization of ethylene, waxes obtained by Fischer-Tropsch synthesis, fatty acid esters and glycerides that are solid at 40 °C, for example, at above 55 °C, silicone waxes such as alkyl- and alkoxy-poly(di)methylsiloxanes and/or poly(di)methylsiloxane esters that are solid at 40 °C, for example, at above 55 °C.

GELLING AGENTS

The compositions of the invention may also be optionally gelled with an oil-phase gelling agent. The gelling agent increases the liquid fatty phase viscosity and leads to a solid or flowable composition when introduced in said fatty phase. The gelling agent does not encompass waxes, in the sense that it is not waxy. The at least one gelling agent may be chosen from gelling agents in polymeric form and gelling agents in mineral form. The gelling agent may be chosen from agents that gel via chemical reticulation and agents that gel via physical reticulation.

Modified clays may be used as gelling agents, examples of which include, but are not limited to, hectorites modified with an ammonium chloride of a $C_{10}$ to $C_{22}$ fatty acid, such as hectorite modified with distearyldimethylammonium chloride, also known as quaternium-18 bentonite, such as the products sold or made under the names Bentone 34 by the company Rheox, Claytone XL, Claytone 34 and Claytone 40 sold or made by the company Southern Clay, the modified clays known under the name quaternium-18 benzalkonium bentonites and sold or made under the names Claytone HT, Claytone GR and Claytone PS by the company Southern Clay, the clays modified with stearyldimethylbenzoylammonium chloride, known as stearalkonium bentonites, such as the products sold or made under the names Claytone APA and Claytone AF by the company Southern Clay, and Baragel 24 sold or made by the company Rheox.

Other mineral gelling agents, which can be used in the invention, include silica, such as fumed silica. The fumed silica may have a particle size, which may be nanometric to micrometric, for example ranging from 5 nm to 200 nm.

The fumed silicas may be obtained by high-temperature hydrolysis of a volatile silicon compound in a hydrogen-oxygen flame, producing a finely divided silica. This process makes it possible to obtain hydrophilic silicas that have a large number of silanol groups at their surface. Such hydrophilic silicas are sold or made, for example, under the names "Aerosil 130®", "Aerosil 200®", "Aerosil 255®", "Aerosil 300®" and "Aerosil 380®" by the company Degussa, and "CAB-O-SIL HS-5®", "CAB-O-SIL EH-5®", "CAB-O-SIL LM-130®", "CAB-O-SIL MS-55®" and "CAB-O-SIL M-5®" by the company Cabot.

It is thus possible to chemically modify the surface of the hydrophilic silica by chemical reaction, producing a reduction in the number of silanol groups. The silanol groups can be replaced, for example, with hydrophobic groups: this then gives a hydrophobic silica. The hydrophobic groups may be: trimethylsiloxyl groups, which are obtained in particular by treating fumed silica in the presence of hexamethyldisilazane. Silicas thus treated are known as "silica silylate" according to the CTFA dictionary. They are sold or made, for example, under the references "Aerosil R812®" by the company Degussa and "CAB-O-SIL TS-530®" by the company Cabot; dimethylsilyloxyl or polydimethylsiloxane groups, which are obtained in particular by treating fumed silica in the presence of polydimethylsiloxane or dimethyldichlorosilane. Silicas thus treated are known as "silica dimethyl silylate" according to the CTFA dictionary. They are sold or made, for example, under the references "Aerosil R972®" and "Aerosil R974®" by the company Degussa, and "CAB-O-SIL TS-610®" and "CAB-O-SIL TS-720®" by the company Cabot; groups derived from reacting fumed silica with silane alkoxides or siloxanes. These treated silicas are, for example, the products sold or made under the reference "Aerosil R805®" by the company Degussa.

According to the invention, hydrophobic silica, such as fumed silica, may be used as a lipophilic gelling agent. The use of fumed silica makes it possible to obtain a translucent or even transparent composition, in particular in the form of a stick, which does not exude, in the absence of opacifying particles such as waxes, fillers and pigments (including

nacres).

The at least one lipophilic gelling agent can allow the exudation of the composition to be limited and can allow its stability to be increased, while at the same time conserving the composition's glossy appearance, which is not possible with waxes such as those used conventionally in cosmetics and dermatology.

The at least one gelling agent, if used, will typically be present in an amount of from 0.1% to 20% by weight, preferably from 0.1% to 15% by weight, and more preferably from 0.1 to 10% by weight, based on the weight of the composition.

ADDITIVES/AUXILIARY AGENTS

The compositions of the present invention may further comprise at least one cosmetically or dermatologically acceptable additive such as a thickener, a film former, a plasticizer, an antioxidant, an essential oil, a preserving agent, a fragrance, a filler, a pasty fatty substance, a waxy fatty substance, a neutralizing agent, and a polymer, and cosmetically active agents and/or dermatological active agents such as, for example, emollients, moisturizers, vitamins, essential fatty acids and medicaments.

While the use of a plasticizer is not necessary in the lip treatment compositions of the present invention, its use may, nevertheless, be desirable. Plasticizers are organic compounds added to a high polymer both to facilitate processing and to increase the flexibility and toughness of the final product by internal modification of the polymer molecule. Examples of suitable plasticizers include, but are not limited to, oils, cellulose esters, phthalate esters, adipate esters, sebacate esters, tricresyl phosphate, castor oil, glycol ethers, benzyl alcohol, triethyl citrate, and propylene carbonate.

Particularly preferred plasticizers include isopropyl palmitate and alkyl benzoate. A plasticizer, if used, will typically be present in an amount of from 1 to 70% by weight, preferably from 2 to 50% by weight, and more preferably from 5 to 20% by weight, based on the weight of the composition.

Representative examples of preservatives include alkyl para-hydroxybenzoates, wherein the alkyl radical has from 1, 2, 3, 4, 5 or 6 carbon atoms and preferably from 1 to 4 carbon atoms e.g., methyl para-hydroxybenzoate (methylparaben), ethyl para-hydroxybenzoate (ethylparaben), propyl para-hydroxybenzoate (propylparaben), butyl para-hydroxybenzoate (butylparaben) and isobutyl para-hydroxybenzoate (isobutylparaben). Mixtures of preservatives may certainly be used, e.g., the mixture of methyl-paraben, ethylparaben, propylparaben and butylparaben sold under the name Nipastat by Nipa, and the mixture of phenoxyethanol, methylparaben, ethylparaben, propylparaben and butylparaben sold under the name Phenonip, also by Nipa. These preservatives may be present in amounts ranging from about 0.01 to about 10% by weight, preferably from 0.5% to about 5% by weight, and more preferably from about 0.8 to about 3% by weight, based on the weight of the composition.

Fillers that may be used in the compositions of the invention include, for example, silica powder; talc; polyamide particles and especially those sold under the name Orgasol by the company Atochem; polyethylene powders; microspheres based on acrylic copolymers, such as those based on ethylene glycol dimethacrylate/lauryl methacrylate copolymer sold by the company Dow Corning under the name Polytrap; expanded powders such as hollow microspheres and especially the microspheres sold under the name Expancel by the company Kemanord Plast or under the name Micropearl F 80 ED by the company Matsumoto; powders of natural organic materials such as crosslinked or noncrosslinked corn starch, wheat starch or rice starch, such as the powders of starch crosslinked with octenyl succinate anhydride, sold under the name Dry-Flo by the company National Starch; silicone resin microbeads such as those sold under the name Tospearl by the company Toshiba Silicone; clays (bentone, laponite, saponite, etc.) and mixtures thereof. These fillers may be present in amounts ranging from about 0.1 to about 50% by weight, preferably from 0.5 to about 30% by weight, and more preferably from about 1 to about 20% by weight, based on the weight of the composition.

The compositions of the present invention may further comprise a safe and effective amount of at least one active ingredient or pharmaceutically acceptable salt thereof. The term "safe and effective amount" as used herein, means an amount sufficient to modify the condition to be treated or to deliver the desired skin benefit, while at the same time avoiding serious side effects, at a reasonable benefit to risk ratio within the scope of sound medical judgment. What is a safe and effective amount of the active ingredient will vary with the specific active agent, the ability of the active agent to penetrate through the skin, the age, health and skin condition of the user, and other like factors. Typically, the active ingredient may be present in amounts ranging from about 0.01 to about 20% by weight, preferably from 0.1 to about 10% by weight, and more preferably from about 0.5 to about 5% by weight, based on the weight of the composition.

The active ingredients useful herein can be categorized by their therapeutic benefit or their postulated mode of action. However, it is to be understood that the active ingredients useful herein can in some instances provide more than one therapeutic benefit or operate via more than one mode of action. Therefore, classifications herein are made for the sake of convenience and are not intended to limit the active ingredient to that particular application or applications listed. Also, pharmaceutically acceptable salts of these active ingredients are useful herein. The following active ingredients are useful in the compositions of the present invention.

Anti-Acne Actives: Examples of useful anti-acne actives include the keratolytics such as salicylic acid (o-hydroxybenzoic acid), derivatives of salicylic acid such as 5-octanoyl salicylic acid, and resorcinol; retinoids such as

retinoic acid and its derivatives (e.g., cis and trans); sulfur-containing D and L amino acids and their derivatives and salts, particularly their N-acetyl derivatives, a preferred example of which is N-acetyl-L-cysteine; lipoic acid; antibiotics and antimicrobials such as benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate, and cholate.

Antimicrobial and Antifungal Actives: Examples of antimicrobial and antifungal actives include .beta.-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, octopirox, parachlorometa xylenol, nystatin, tolnaftate and clotrimazole.

The cosmetic compositions of this invention may also contain sunscreens, which are chemical absorbers that actually absorb harmful ultraviolet radiation. It is well known that chemical absorbers are classified, depending on the type of radiation they protect against, as either UV-A or UV-B absorbers. UV-A absorbers generally absorb radiation in the 320 to 400 nm region of the ultraviolet spectrum. UV-A absorbers include anthranilates, benzophenones, and dibenzoyl methanes. UV-B absorbers generally absorb radiation in the 280 to 320 nm region of the ultraviolet spectrum. UV-B absorbers include *p*-aminobenzoic acid derivatives, camphor derivatives, cinnamates, and salicylates.

The sunscreens useful in the present invention typically comprise chemical absorbers, but may also comprise physical blockers. Exemplary sunscreens which may be formulated into the compositions of the present invention are chemical absorbers such as p-aminobenzoic acid derivatives, anthranilates, benzophenones, camphor derivatives, cinnamic derivatives, dibenzoyl methanes (such as avobenzone also known as Parsol® 1789), diphenylacrylate derivatives, salicylic derivatives, triazine derivatives, benzimidazole compounds, bis-benzoazolyl derivatives, methylene bis-(hydroxyphenylbenzotriazole) compounds, the sunscreen polymers and silicones, or mixtures thereof. Also exemplary of the sunscreens which may be formulated into the compositions of this invention are physical blockers such as cerium oxides, chromium oxides, cobalt oxides, iron oxides, red petrolatum, silicone-treated titanium dioxide, titanium dioxide, zinc oxide, and/or zirconium oxide, or mixtures thereof.

Examples of suitable sunscreens include, but are not limited to: aminobenzoic acid, amyldimethyl PABA, cinoxate, diethanolamine *p*-methoxycinnamate, digalloyl trioleate, dioxybenzone, 2-ethoxyethyl *p*-methoxycinnamate, ethyl 4-bis(hydroxypropyl)aminobenzoate, 2-ethylhexyl-2-cyano-3,3-diphenylacrylate, ethylhexyl *p*-methoxycinnamate, 2-ethylhexyl salicylate, glyceryl aminobenzoate, homomenthyl salicylate, homosalate, 3-imidazol-4-ylacrylic acid and ethyl ester, methyl anthranilate, octyldimethyl PABA, 2-phenylbenzimidazole-5-sulfonic acid and salts, red petrolatum, sulisobenzone, titanium dioxide, triethanolamine salicylate, *N*, *N*, *N*-trimethyl-4-(2-oxoborn-3-ylidene methyl)anillinium methyl sulfate, and mixtures thereof.

The lip treatment composition of the invention may be in the form of a lipstick, a lip gloss or a lip pencil, optionally having care or treating properties.

RHEOLOGY

The rheological properties of the compositions in accordance with the present invention are determined by using a controlled stress rheometer, commercially available from TA Instruments under the name AR-G2. The samples are measured using a parallel plate having a stainless steel, cross hatched, 40 mm diameter plate. The gap is set at 1,000 microns. The desired temperature is precisely controlled by a Peltier system.

The lip-gloss sample is transferred to the rheometer, and heated to 35°C for about 10 minutes. The sample is then cooled and held at 25 °C for about 10 minutes or more.

The linear viscoelastic regime is determined by oscillation stress sweep mode with a range of from 1 mN.m to 100 mN.m, at a constant frequency of 1 rad/s. Said linear viscoelastic regime corresponds to the elastic/storage modulus G', within the above range, when the elastic/storage modulus G' is constant, or nearly constant, at the applied oscillation stress.

The frequency sweep experiment is then performed from 100 rad/s to 0.01 rad/s at a low oscillation stress in the linear viscoelastic regime. The elastic/storage modulus G' at a frequency $\omega$ of 0.01 rad/s is determined from the frequency sweep mode.

The lower the value of the elastic/storage modulus G', at a frequency $\omega$ of 0.01 rad/s, the better the wetting

property and the less creep resistance for the lip-gloss composition.

In the linear viscoelastic regime, the elastic/storage modulus G' at a frequency $\omega$ of 0.01 rad/s, of compositions in accordance with the present invention, is in the range of from 0.01 Pa to 500 Pa at 25 °C.

After finishing the dynamic oscillation experiment, the same sample is equilibrated for 10 minutes, at a constant temperature of 25°C. Creep and recovery measurements are then performed at a constant stress of 0.8 Pa.

The creep viscosity ($\eta_{creep}$) of the lip gloss composition, measured at a constant stress ($\sigma$) of 0.8 Pa, is determined from the creep strain ($\gamma_{creep}$) and the recoverable strain ($\gamma_{recovery}$), wherein the creep strain duration ($t_{creep}$) is 10 minutes and the recoverable strain duration is 30 minutes. The creep viscosity is calculated by the following expression:

$$\eta_{creep} = \frac{\sigma\, t_{creep}}{\left(\gamma_{creep}(t = 10\,\mathbf{min}) - \gamma_{recovery}(t = 30\,\mathbf{min})\right)}$$

A high creep viscosity value ($\eta_{creep}$) at low stress, with a creep time of 10 minutes (near zero shear rate), provides for a longer wear of the composition. Therefore, a lip composition with a high creep viscosity value ($\eta_{creep}$) at low stress will maintain its structure, thus its stability, at rest, will show less migration, and will provide a lasting shine.

The creep viscosity ($\eta_{creep}$) of compositions in accordance with the present invention, at a constant stress ($\sigma$) of 0.8 Pa, is in the range of from 2 Pa.s to 150,000 Pa.s at 25 °C.

METHOD OF MAKING-UP

TOPCOAT COMPOSITION

As stated previously, the composition according to the present invention may be advantageously used as a topcoat in the claimed method of making-up according to the invention.

This composition is as defined previously.

Furthermore, it may be desirable to enhance the comfort of the topcoat composition on the keratinous substrate onto which it is applied such as, for example, lips. This can be achieved by the use of a short chain ester.

According to the invention, the esters may either be monoesters, diesters or polyesters. These esters may be linear, branched or cyclic, saturated or unsaturated. These esters should preferably be branched and saturated. They may also be aliphatic or aromatic.

These esters may have from 6 to 25 carbon atoms and particularly from 14 to 22 carbon atoms. They may be chosen amongst acid esters having from 2 to 18 carbon atoms, and particularly amongst alcohol esters having from 2 to 20 carbon atoms or amongst polyols having from 2 to 8 carbon atoms or their mixtures, on condition that the number of carbon atoms is higher than 10, so that the ester is not volatile and penetrates the skin.

Particularly, these esters are hydrocarbon-based esters which correspond to the following formula RCOOR' where R represents a residue of fatty acid having from 1 to 29 carbon atoms, and R' represents a hydrocarbon-based chain containing from 2 to 30 carbon atoms, on condition that the number of carbon atoms in R' is higher than 10, so that the ester is not volatile and penetrates the skin.

The ester may be chosen among a non-limitative list including the following:

Neopentanoic acid esters such as isodecyl neopentanoate, isotridecyl neopentanoate, isostearyl neopentanoate, octyldocecyl neopentanoate,

Isononanoic acid esters such as isononyl isononanoate, octyl isononanoate, isodecyl isononanoate, isotridecyl isononanoate, isostearyl isononanoate, ethylhexyl isononanoate,

Isopropylic alcohol esters, such as isopropyl myristate, isopropyl palmitate, isopropyl stearate or isostearate, isopropyl laurate, diisopropyl adipate,

Alkyl or polyalkyl octanoates, decanoates or ricinoleates, such as cetyl octanoate, tridecyl octanoate,

polyalkylene glycol esters, such as polyethylene glycol diheptanoate, hexanoate-2-diethyl propylene glycol and their mixtures,

Benzoate alkyls particularly benzoate alkyls having from 12 to 15 carbon atoms,

Hydroxylated esters such as isotearyl lactate and diisostearyl malate, and

Pentaexythritol esters.

Examples of short chain esters also include purcellin oil (cetostearyl octanoate), ethylhexyl ethylhexanoate, dicapryl ester, 2-ethylhexyl palmitate, 2-ethyl-palmitate and isostearyl isostearate.

The isononyl isononanoate and diisostearyl malate are particularly suited for the embodiment of this invention.

The short chain ester(s) may be used in the topcoat composition in an amount of up to about 15% by weight, preferably up to about 10% by weight, based on the weight of the topcoat composition. It should be noted that the use of too much short chain ester(s) in the topcoat will have a deleterious effect on the non-transfer properties of the cosmetic product system as it will cause the topcoat to become too compatible with the basecoat.

BASECOAT COMPOSITION

"Film former" or "film forming agent" as used herein means a polymer that, after dissolution in at least one solvent (such as, for example, water and organic solvents), leaves a film on the substrate to which it is applied, for example, once the at least one solvent evaporates, absorbs and/or dissipates on the substrate.

"Transfer resistance" as used herein refers to the quality exhibited by compositions that are not readily removed by contact with another material, such as, for example, a glass, an item of clothing or the skin, for example, when eating or drinking. Transfer resistance may be evaluated by any method known in the art for evaluating such. For example, transfer resistance of a composition may be evaluated by a "kiss" test. The "kiss" test may involve application of the composition to human lips followed by "kissing" a material, for example, a sheet of paper, after expiration of a certain amount of time following application, such as 2 minutes after application. Similarly, transfer resistance of a composition may be evaluated by the amount of product transferred from a wearer to any other substrate, such as transfer from the neck of an individual to a collar after the expiration of a certain amount of time following application. The amount of composition transferred to the substrate (e.g., collar, or paper) may then be evaluated and compared. For example, a composition may be transfer resistant if a majority of the product is left on the wearer, e.g., lips, neck, etc. Further, the amount transferred may be compared with that transferred by other compositions, such as commercially available compositions. In a preferred embodiment of the present invention, little or no composition is transferred to the substrate.

"Long wear" compositions as used herein, refers to compositions where at least one property chosen from consistency, texture, and color remains the same as at the time of application, as viewed by the naked eye, after an extended period of time, such as, for example, 1 hour, 2 hours, and further such as 8 hours. Long wear properties may be evaluated by any method known in the art for evaluating such properties. For example, long wear may be evaluated by a test involving the application of a composition to human skin (including lips) and evaluating the consistency, texture and color of the composition after an extended period of time. For example, the consistency, texture and color of a lip composition may be evaluated immediately following application and these characteristics may then be reevaluated and compared after an individual has worn the lip composition for a certain amount of time. Further, these characteristics may be evaluated with respect to other compositions, such as commercially available compositions.

"Waterproof" as used herein refers to the ability to repel water and permanence with respect to water. Waterproof properties may be evaluated by any method known in the art for evaluating such properties. For example, a mascara composition may be applied to false eyelashes, which may then be placed in water for a certain amount of time, such as, for example, 20 minutes. Upon expiration of the pre-ascertained amount of time, the false eyelashes may be removed from the water and passed over a material, such as, for example, a sheet of paper. The extent of residue left on the material may then be evaluated and compared with other compositions, such as, for example, commercially available compositions. Similarly, for example, a composition may be applied to skin, and the skin may be submerged in water for a certain amount of time. The amount of composition remaining on the skin after the pre-ascertained amount of time may then be evaluated and compared. For example, a composition may be waterproof if a majority of the product is left on the wearer, e.g., eyelashes, skin, etc. In a preferred embodiment of the present invention, little or no composition is transferred from the wearer.

The cosmetic compositions and methods of the present invention can comprise, consist of, or consist essentially of the essential elements and limitations of the invention described herein, as well as any additional or optional ingredients, components, or limitations described herein or otherwise useful in personal care compositions intended for topical application to the skin.

In accordance with certain aspects of the present invention, the phrase "liquid fatty phase" is understood to mean a fatty phase, which is liquid at room temperature (25°C) and atmospheric pressure (760 mmHg), and which comprises one or more fatty substances that are liquid at room temperature, also known as oils, which are compatible with one another.

In accordance with certain aspects of the present invention, the phrase "structured liquid fatty phase" is understood to mean that this structured phase does not run between the fingers and is at least thickened.

Where the liquid fatty phase is structured, it makes it possible to limit exudation of the fatty phase from solid compositions, and furthermore, to limit, after deposition on the skin or the lips, its migration into the wrinkles and fine lines, which is desired for compositions such as a lipstick or an eyeshadow. Significant migration of the liquid fatty phase, laden with coloring materials, leads to an unaesthetic effect around the lips or the eyes, which can accentuate the wrinkles and fine lines. This migration is often mentioned by women as being a major defect of conventional lipsticks and eyeshadows. The term "migration" is understood to mean running of the composition deposited on the lips or skin beyond its initial outline.

"Gloss" is essentially related to the nature of the liquid fatty phase. Thus, it is possible to reduce the level of waxes and fillers in the composition in order to increase the gloss of a lipstick, but then the migration of the liquid fatty phase increases. In other words, the levels of waxes and/or of fillers necessary for preparation of a stick of suitable hardness have been a restricting factor on the gloss of the deposit.

"Tackiness" as used herein refers to measuring the maximum tensile force, $F_{max}$, required while separating

two surfaces. Depending on the application envisaged and the formulation being designed, the desirable value for $F_{max}$ may vary. In some embodiments, the substantially non-tacky compositions have a $F_{max}$ of less than about 4 Newton (N), less than about 1 N, less than about 0.5 N, less than about 0.3 N, less than about 0.2 N or less than 0.1 N. One of ordinary skill in the art can determine the $F_{max}$ of the composition by, for example, determining the maximum force of traction, measured with an extensiometer of the LLOYD model LR5K type, needed to detach two surfaces.

For example, two 38 $mm^2$ surfaces, A and B, which are solid, rigid, inert, and non-absorbing, are mounted on movable mounts, facing each other. The surfaces may be movable either toward or away from each other, or one may move surface A independently from surface B or vice versa. Prior to insertion into the extensiometer, surface A is coated with the composition to be measured, which may be dissolved in a solvent such as aqueous, hydroalcoholic, hydrocarbon, silicone, and alcoholic solvents in a concentration of from about 10 to about 30%, preferably 20%, the surface A is coated in a thickness of from 1 to 10 mil, preferably 1 mil, and the surface is dried for 24 hours at room temperature, e.g., 22 to 25°C, at a relative humidity of about 50%. Once inserted in the extensiometer, surface A is subjected for 20 seconds to a compression force of 3 N against surface B and then subjected for 30 seconds to tensile force at a rate of 20 mm/minute. The amount of force, $F_{max}$, needed to obtain initial separation is then noted. A mean $F_{max}$ is determined by carrying out the procedure with multiple pairs, preferably at least six pairs, of surface A and surface B.

The cosmetic product system of the present invention may be in any form. For example, it may be a paste, a solid, a gel, or a cream. It may be an emulsion, such as an oil-in-water or water-in-oil emulsion, a multiple emulsion, such as an oil-in-water-in-oil emulsion or a water-in-oil-in-water emulsion, or a solid, rigid or supple gel, including anhydrous gels. The system can also be in a form chosen from a translucent anhydrous gel and a transparent anhydrous gel. The system of the invention may, for example, comprise an external or continuous fatty phase. The system may be anhydrous. In another embodiment, the system of the invention may be transparent or clear, including for example, a composition without pigments. The system can also be a molded composition or cast as a stick or a dish. The compositions in one embodiment are solid such as a molded stick or a poured stick. The compositions of the present invention may also be in the form a lip composition such as a lipstick or a liquid lip color, a foundation or a mascara, which exhibit excellent and improved properties of transfer-resistance, flexibility, pliability, adherence and lack of tackiness.

Where the composition of the invention is not-liquid, the structuring of the liquid fatty phase can be controlled by the type of polyorganosiloxane-containing polymer (or structuring polymer) used and is such that a rigid structure in the form of a stick, of good mechanical resistance, can be obtained. These rigid compositions, when colored, allow for a supple, light, non-transfer, non-migrating and/or long-wearing applications on a keratinous surface. Such compositions may contain one or more structuring polymers.

As defined herein, stability is tested by placing the composition in a controlled environment chamber for 8 weeks at 25°C. In this test, the physical condition of the sample is inspected as it is placed in the chamber. The sample is then inspected again at 24 hours, 3 days, 1 week, 2 weeks, 4 weeks and 8 weeks. At each inspection, the sample is examined for abnormalities in the composition such as phase separation if the composition is in the form of an emulsion, bending or leaning if the composition is in stick form, melting, or syneresis (or sweating). The stability is further tested by repeating the 8-week test at 40°C, 37°C, 45°C, 50°C and under freeze-thaw conditions. A composition is considered to lack stability if in any of these tests an abnormality that impedes functioning of the composition is observed. The skilled artisan will readily recognize an abnormality that impedes functioning of a composition based on the intended application.

Polyorganosiloxane containing polymer

According to the present invention, compositions comprising at least one polyorganosiloxane containing polymer chosen from homopolymers and copolymers, preferably, with a weight-average molecular mass ranging from about 500 to about 2.5 x $10^6$ or more, comprising at least one moiety comprising: at least one polyorganosiloxane group comprising, preferably, from 1 to about 10,000 organosiloxane units in the chain of the moiety or in the form of a graft, and at least two groups capable of establishing hydrogen interactions are provided.

According to preferred embodiments of the present invention, the polyorganosiloxane-containing polymers used in the composition of the invention may belong to the following two families:

a) polyorganosiloxanes comprising at least two groups capable of establishing hydrogen interactions, these two groups being located in the polymer chain; and/or

b) polyorganosiloxanes comprising at least two groups capable of establishing hydrogen interactions, these two groups being located on grafts or branches.

The polyorganosiloxane containing polymers of the present invention can be liquid or solid at room temperature. Preferably, the polymers are solid. When the polymers are solid, it is preferable that they can be dissolved before or during use in a solvent with hydrogen interaction capable of breaking the hydrogen interactions of the polymers, for instance $C_2$ to $C_8$ lower alcohols and especially ethanol, n-propanol or isopropanol. It is also possible to use these

hydrogen interaction "breaking" solvents as co-solvents in the compositions of the present invention. These solvents may then be stored in the composition or may be removed by selective evaporation, which is well known to those skilled in the art.

The polymers comprising two groups capable of establishing hydrogen interactions in the polymer chain may be polymers comprising at least one moiety corresponding to the formula:

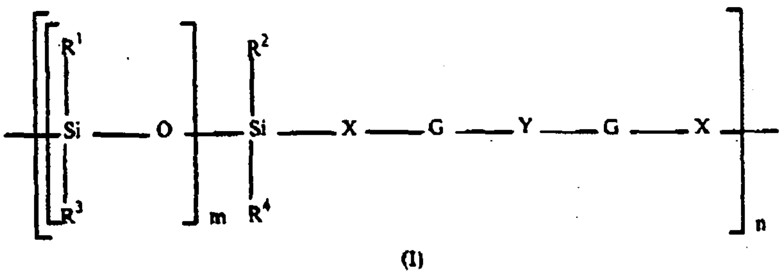

(I)

in which:

1) $R^1$, $R^2$, $R^3$ and $R^4$, which may be identical or different, represent a group chosen from:

- linear, branched or cyclic, saturated or unsaturated, $C_1$ to $C_{40}$ hydrocarbon-based groups, possibly containing in their chain one or more oxygen, sulphur and/or nitrogen atoms, and possibly being partially or totally substituted with fluorine atoms,
- $C_6$ to $C_{10}$ aryl groups, optionally substituted with one or more $C_1$ to $C_4$ alkyl groups,
- polyorganosiloxane chains possibly containing one or more oxygen, sulphur and/or nitrogen atoms;

2) the groups x, which may be identical or different, represent a linear or branched $C_1$ to $C_{30}$ alkylenediyl group, possibly containing in its chain one or more oxygen and/or nitrogen atoms;
3) Y is a saturated or unsaturated, $C_1$ to $C_{50}$ linear or branched divalent alkylene, arylene, cycloalkylene, alkylarylene or arylalkylene group, possibly comprising one or more oxygen, sulphur and/or nitrogen atoms, and/or bearing as substituent one of the following atoms or groups of atoms: fluorine, hydroxyl, $C_3$ to $C_8$ cycloalkyl, $C_1$ to $C_{40}$ alkyl, $C_5$ to $C_{10}$ aryl, phenyl optionally substituted with 1 to 3 $C_1$ to $C_3$ alkyl groups, $C_1$ to $C_3$ hydroxyalkyl and $C_1$ to $C_6$ aminoalkyl, or
4) Y represents a group corresponding to the formula :

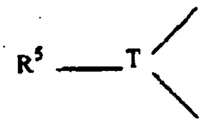

in which

- T represents a linear or branched, saturated or unsaturated, $C_3$ to $C_{24}$ trivalent or tetravalent hydrocarbon-based group optionally substituted with a polyorganosiloxane chain, and possibly containing one or more atoms chosen from O, N and S, or T represents a trivalent atom chosen from N, P and A1, and
- $R^5$ represents a linear or branched $C_1$ to $C_{50}$ alkyl group or a polyorganosiloxane chain, possibly comprising one or more ester, amide, urethane, thiocarbamate, urea, thiourea and/or sulphonamide groups, which may be linked to another chain of the polymer;

5) the groups G, which may be identical or different, represent divalent groups chosen from:

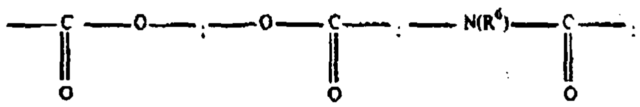

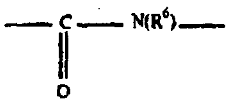 ; 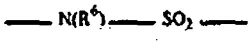 ; 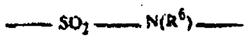 ;

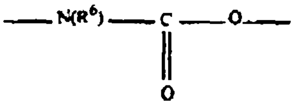 ; 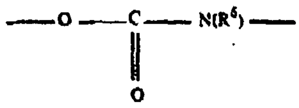 ; 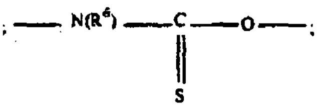 ;

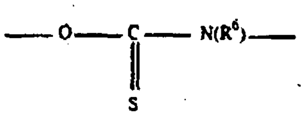 ; 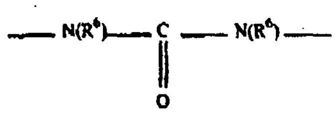 and

and

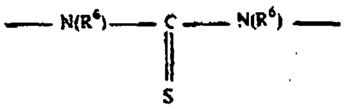

in which $R^6$ represents a hydrogen atom or a linear or branched $C_1$ to $C_{20}$ alkyl group, on condition that at least 50% of the groups $R^6$ of the polymer represents a hydrogen atom and that at least two of the groups G of the polymer are a group other than:

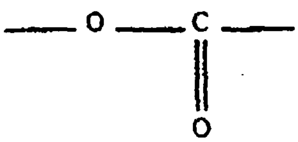 and 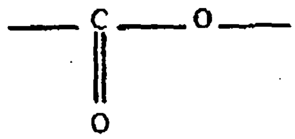 ;

6) n is an integer of at least 1, for example ranging from 2 to 500 and preferably from 2 to 200, and m is an integer of at least one, ranging from 1 to 35,000, for example, from 1 to 10,000 and 1 to 2,500, from 1 to 700 and from 6 to 200, including all values and subranges there between.

According to the invention, 80% of the groups $R^1$, $R^2$, $R^3$ and $R^4$ of the polymer are preferably chosen from methyl, ethyl, phenyl and 3,3,3-trifluoropropyl groups.

According to the invention, Y can represent various divalent groups, furthermore optionally comprising one or two free valencies to establish bonds with other moieties of the polymer or copolymer. Preferably, Y represents a group chosen from:

a) linear $C_1$ to $C_{20}$ and preferably $C_1$ to $C_{10}$ alkylene groups,
b) $C_{30}$ to $C_{56}$ branched alkylene groups possibly comprising rings and unconjugated unsaturations,
c) $C_5$-$C_6$ cycloalkylene groups,
d) phenylene groups optionally substituted with one or more $C_1$ to $C_{40}$ alkyl groups,
e) $C_1$ to $C_{20}$ alkylene groups comprising from 1 to 5 amide groups,
f) $C_1$ to $C_{20}$ alkylene groups comprising one or more substituents chosen from hydroxyl, $C_3$ to $C_8$ cycloalkane, $C_1$ to $C_3$ hydroxyalkyl and $C_1$ to $C_6$ alkylamine groups,
g) polyorganosiloxane chains of formula:

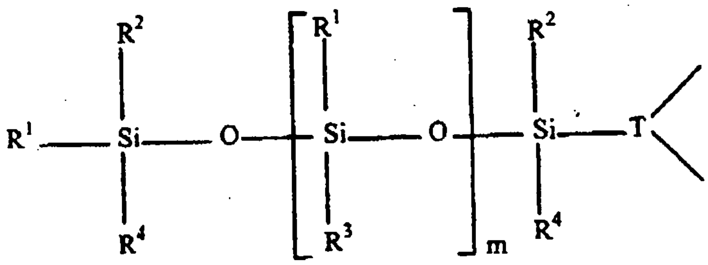

in which $R^1$, $R^2$, $R^3$, $R^4$, T and m are as defined above, and

h) polyorganosiloxane chains of formula:

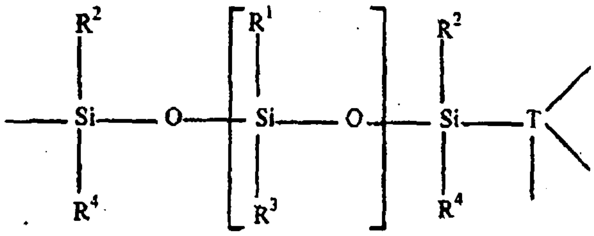

The polyorganosiloxanes of the second family may be polymers comprising at least one moiety corresponding to formula (II)

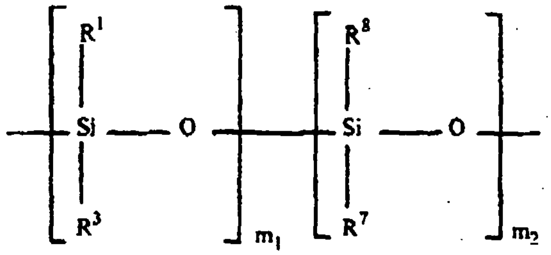 (II)

in which

- $R^1$ and $R^3$, which may be identical or different, are as defined above for formula (I),
- $R^7$ represents a group as defined above for $R^1$ and $R^3$, or represents a group of formula -X-G-$R^9$ in which X and G are as defined above for formula (I) and $R^9$ represents a hydrogen atom or a linear, branched or cyclic, saturated or unsaturated, $C_1$ to $C_{50}$ hydrocarbon-based group optionally comprising in its chain one or more atoms chosen from O, S and N, optionally substituted with one or more fluorine atoms and/or one or more hydroxyl groups, or a phenyl group optionally substituted with one or more $C_1$ to $C_4$ alkyl groups,
- $R^8$ represents a group of formula -X-G-$R^9$ in which X, G and $R^9$ are as defined above,
- $m_1$ is an integer of at least one ranging from 1 to 35,000, for example, from 1 to 10,000 and 1 to 2,500, from 1 to 700, and from 6 to 200, including all values and subranges there between; and
- $m_2$ is an integer of at least one ranging from 1 to 35,000, for example, from 1 to 10,000 and 1 to 2,500, from 1 to 700, and from 6 to 200, including all values and subranges there between.

According to the invention, the polyorganosiloxane containing polymer may be a homopolymer, that is to say a polymer comprising several identical moieties, in particular moieties of formula (I) or of formula (II).

According to the invention, it is also possible to use a polymer consisting of a copolymer comprising several different moieties of formula (I), that is to say a polymer in which at least one of the groups $R^1$, $R^2$, $R^3$, $R^4$, X, G, Y, m and n is different in one of the moieties. The copolymer may also be formed from several moieties of formula (II), in which at least one of the groups $R^1$, $R^3$, $R^7$, $R^8$, $m_1$ and $m_2$ is different in at least one of the moieties.

It is also possible to use a copolymer comprising at least one moiety of formula (I) and at least one moiety of

formula (II), the moieties of formula (I) and the moieties of formula (II) possibly being identical to or different from each other.

According to preferred embodiments, it is also possible to use a copolymer comprising at least one hydrocarbon-based moiety comprising two groups capable of establishing hydrogen interactions, chosen from ester, amide, sulphonamide, carbamate, thiocarbamate, urea and thiourea groups, and combinations thereof.

According to a first embodiment of the invention, the groups capable of establishing hydrogen interactions are amide groups of formulae -C(O)NH- and -HN-C(O)-.

These copolymers may be block copolymers or grafted copolymers.

In this case, the polymer may comprise at least one moiety of formula (III) or (IV)

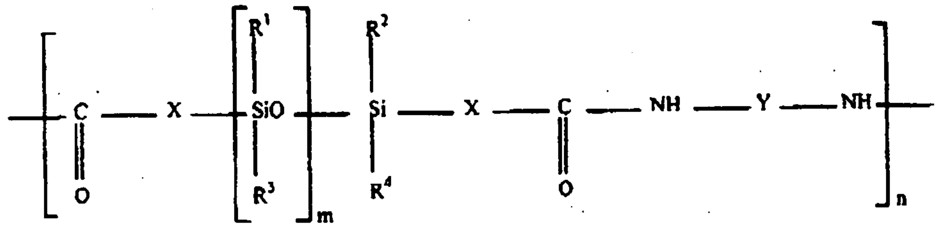

(III)

or

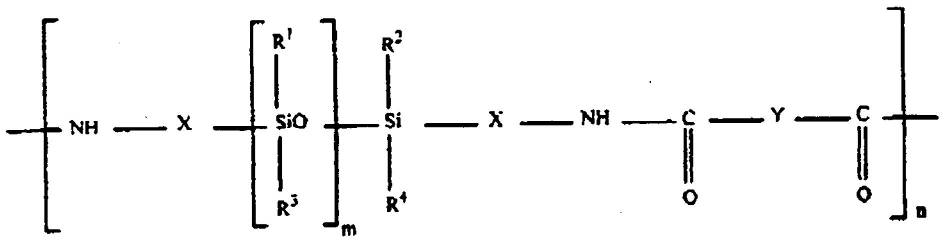

(IV)

in which $R^1$, $R^2$, $R^3$, $R^4$, X, Y, m and n are as defined above.

Such a moiety may be obtained :

- either by a condensation reaction between a silicone containing α, ω-carboxylic acid ends and one or more diamines, according to the following reaction scheme:

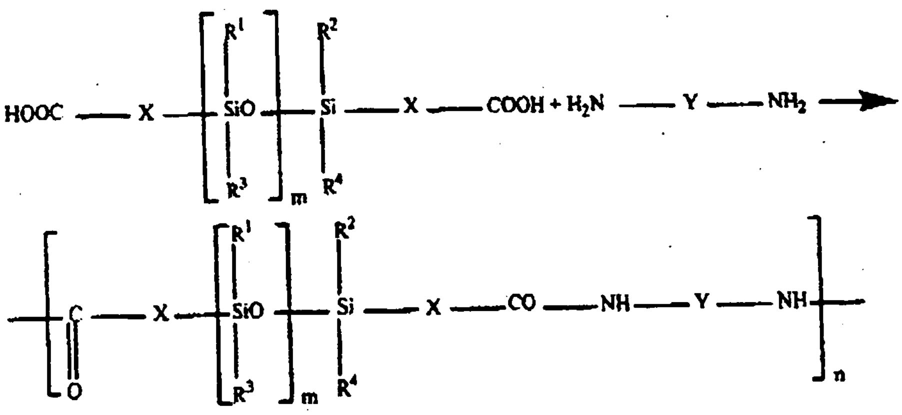

- or by reaction of two molecules of α-unsaturated carboxylic acid with a diamine according to the following reaction scheme:

$$CH_2\text{-}CH\text{-}X^1\text{-}COOH + H_2N\text{-}Y\text{-}NH_2 \rightarrow CH_2\text{=}CH\text{-}X^1\text{-}CO\text{-}NH\text{-}Y\text{-}NH\text{-}CO\text{-}X^1\text{-}CH\text{=}CH_2$$

followed by the addition of a siloxane to the ethylenic unsaturations, according to the following scheme: $CH_2=CH-X^1-CO-NH-Y-NH-CO-X^1-CH=CH_2$

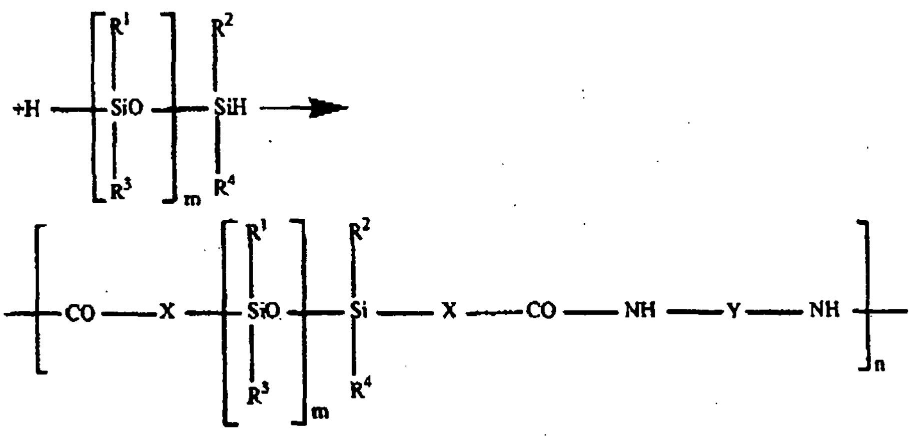

in which $X^1-(CH_2)_2-$ corresponds to X defined above and Y, $R^1$, $R^2$, $R^3$, $R^4$ and m are as defined above ;

- or by reaction of a silicone containing α,ω-$NH_2$ ends and a diacid of formula HOOC-Y-COOH according to the following reaction scheme:

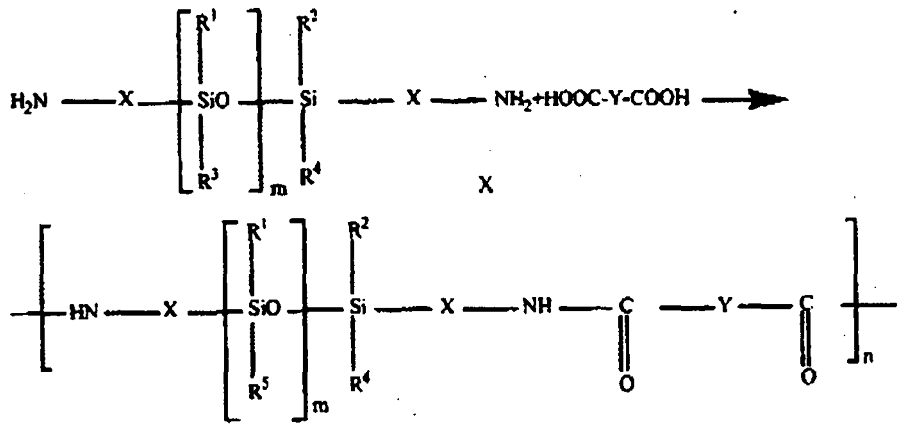

In these polyamides of formula (III) or (IV), m is an integer of at least one as defined above, and preferably in the range from 1 to 700, for example, from 15 to 500 and from 15 to 45, including all values and subranges there between; and n is in particular in the range from 1 to 500, for example, from 1 to 100 and from 4 to 25, including all values and subranges there between; X is preferably a linear or branched alkylene chain containing from 1 to 30 carbon atoms and in particular 3 to 10 carbon atoms, and Y is preferably an alkylene chain that is linear or branched or that possibly comprises rings and/or unsaturations, containing from 1 to 40 carbon atoms, including from 1 to 20 carbon atoms and from 2 to 6 carbon atoms, including all values and subranges there between, for example, 6 carbon atoms.

In formulae (III) and (IV), the alkylene group representing X or Y can optionally contain in its alkylene portion at least one of the following elements:

1) 1 to 5 amide, urea or carbamate groups,
2) a $C_5$ or $C_6$ cycloalkyl group, and
3) a phenylene group optionally substituted with 1 to 3 identical or different $C_1$ to $C_3$ alkyl groups.

In formulae (III) and (IV), the alkylene groups may also be substituted with at least one element chosen from the group consisting of:

- a hydroxyl group,
- a $C_3$ to $C_8$ cycloalkyl group,
- one to three $C_1$ to $C_{40}$ alkyl groups,
- a phenyl group optionally substituted with one to three $C_1$ to $C_3$ alkyl groups,
- a $C_1$ to $C_3$ hydroxyalkyl group, and
- a $C_1$ to $C_6$ aminoalkyl group.

In these formulae (III) and (IV), Y may also represent:

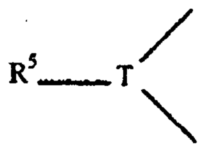

in which $R^5$ represents a polyorganosiloxane chain and T represents a group of formula:

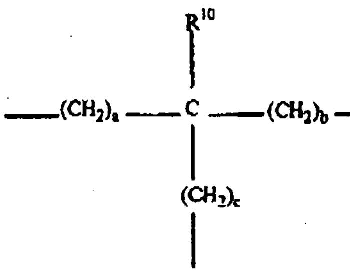

or

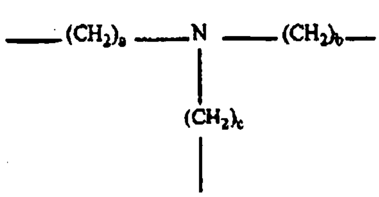

in which a, b and c are, independently, integers ranging from 1 to 10, and $R^{10}$ is a hydrogen atom or a group such as those defined for $R^1$, $R^2$, $R^3$ and $R^4$.

In formulae (III) and (IV), $R^1$, $R^2$, $R^3$ and $R^4$ preferably represent, independently, a linear or branched $C_1$ to $C_{40}$ alkyl group, preferably a $CH_3$, $C_2H_5$, n-$C_3H_7$ or isopropyl group, a polyorganosiloxane chain or a phenyl group optionally substituted with one to three methyl or ethyl groups.

As has been seen previously, the polymer may comprise identical or different moieties of formula (III) or (IV).

Thus, the polymer may be a polyamide containing several moieties of formula (III) or (IV) of different lengths, i.e. a polyamide corresponding to the formula :

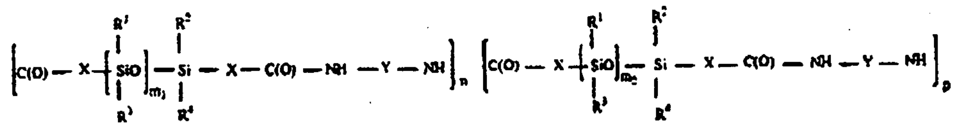

(V)

in which X, Y, n and $R^1$ to $R^4$ have the meanings given above, $m_1$ and $m_2$, which are different, are as defined above, and preferably are chosen in the range from 1 to 1 000, and p is at least one for example ranging from 2 to 500 and preferably from 2 to 200.

In this formula, the moieties may be structured to form either a block copolymer, or a random copolymer or an alternating copolymer. In this copolymer, the moieties may be not only of different lengths, but also of different chemical structures, for example containing different groups Y. In this case, the copolymer may correspond to the formula :

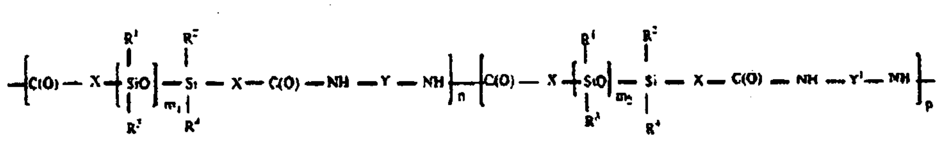

(VI)

in which $R^1$ to $R^4$, X, Y, $m_1$, $m_2$, n and p have the meanings given above and $Y^1$ is different from Y but chosen from the groups defined for Y. As previously discussed, the various moieties may be structured to form either a block copolymer, or a random copolymer or an alternating copolymer.

In an embodiment of the invention, the polyorganosiloxane-containing polymer may also contain a grafted copolymer. Thus, the polyamide containing silicone units may be grafted and optionally crosslinked with silicone chains containing amide groups. Such polymers may be synthesized with trifunctional amines.

In this case, the copolymer may comprise at least one moiety of formula :

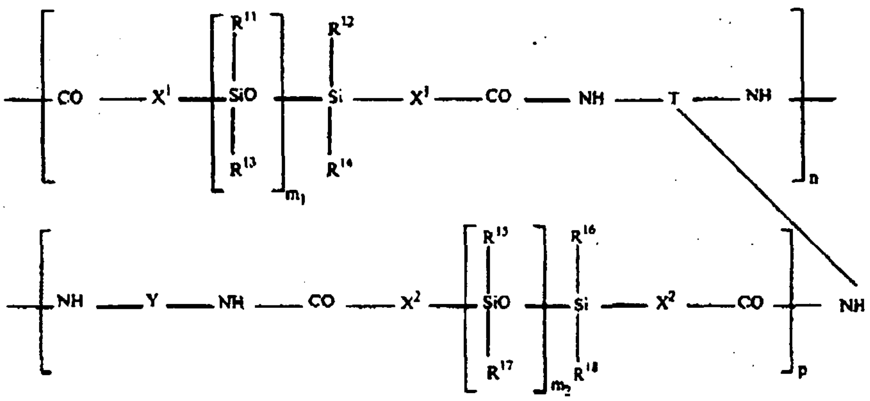

(VII)

in which $X^1$ and $X^2$, which may be identical or different, have the meaning given for X in formula (I), n is as defined in formula (I), Y and T are as defined in formula (I), $R^{11}$ to $R^{18}$ are groups chosen from the same group as $R^1$ to $R^4$, $m_1$ and $m_2$ are numbers in the range from 1 to 1,000, and p is an integer of at least one, for example, p can range from 2 to 500.

In formula (VII), it is preferred that :

- p is in the range from 1 to 25, including from 1 to 7, including a11 values and subranges there between,
- $R^{11}$ to $R^{18}$ are methyl groups,
- T corresponds to one of the following formulae:

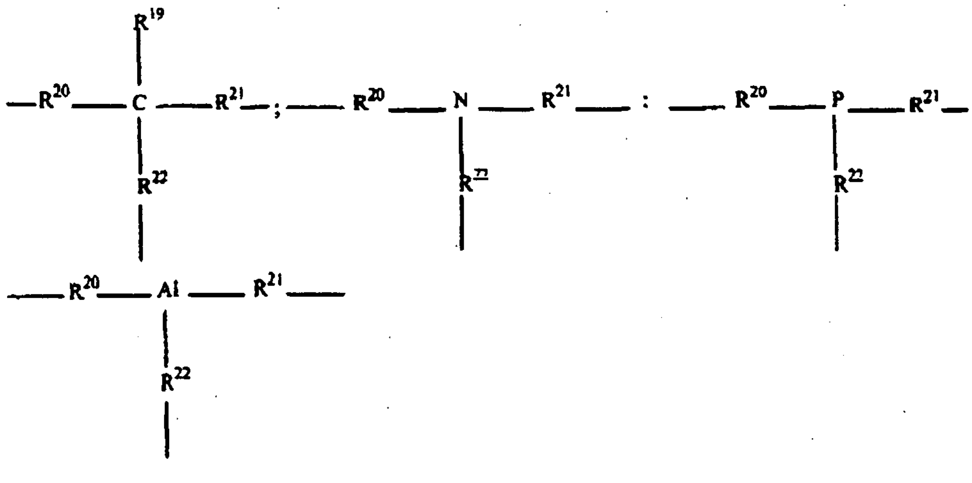

in which $R^{19}$ is a hydrogen atom or a group chosen from the groups defined for $R^1$ to $R^4$, and $R^{20}$, $R^{21}$ and $R^{22}$ are, independently, linear or branched alkylene groups, and more preferably corresponds to the formula:

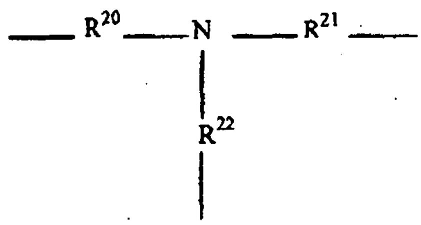

in particular with $R^{20}$, $R^{21}$ and $R^{22}$ representing $-CH_2-CH_2-$,

- $m_1$ and $m_2$ are in the range from 15 to 500, including from 15 to 45 and including all values and subranges there between,
- $X^1$ and $X^2$ represent $-(CH_2)_{10}-$, and
- Y represents $-CH_2-$.

These polyamides containing a grafted silicone moiety of formula (VII) may be copolymerized with polyamide-silicones of formula (II) to form block copolymers, alternating copolymers or random copolymers. The weight percentage of grafted silicone moieties (VII) in the copolymer may range from 0.5% to 30% by weight.

According to the invention, as has been seen previously, the siloxane units may be in the main chain or backbone of the polymer, but they may also be present in grafted or pendent chains. In the main chain, the siloxane units may be in the form of segments as described above. In the pendent or grafted chains, the siloxane units may appear individually or in segments.

According to the invention, the preferred siloxane-based polyamides are :

- polyamides of formula (III) in which m is from 15 to 300, for example, 15 to 100, including all values and subranges there between;
- mixtures of two or more polyamides in which at least one polyamide has a value of m in the range from 15 to 50, including all values and subranges there between and at least one polyamide has a value of m in the range from 30 to 300, including all values and subranges there between;
- polymers of formula (V) with $m_1$ chosen in the range from 15 to 50 and $m_2$ chosen in the range from 30 to 500 with the portion corresponding to $m_1$ representing 1% to 99% by weight of the total weight of the polyamide and the corresponding portion $m_2$ representing 1% to 99% by weight of the total weight of the polyamide;
- mixtures of polyamide of formula (III) combining

  1) 80% to 99% by weight of a polyamide in which n is equal to 2 to 10 and in particular 3 to 6, and
  2) 1% to 20% of a polyamide in which n is in the range from 5 to 500 and in particular from 6 to 100;

- polyamides corresponding to formula (VI) in which at least one of the groups Y and $Y^1$ contains at least one hydroxyl substituent;
- polyamides of formula (III) synthesized with at least one portion of an activated diacid (diacid chloride, dianhydride or diester) instead of the diacid;
- polyamides of formula (III) in which X represents $-(CH_2)_3-$ or $-(CH_2)_{10}$; and
- polyamides of formula (III) in which the polyamides end with a monofunctional chain chosen from the group consisting of monofunctional amines, monofunctional acids, monofunctional alcohols, including fatty acids, fatty alcohols and fatty amines, such as, for example, octylamine, octanol, stearic acid and stearyl alcohol.

According to the invention, the end groups of the polymer chain may end with :

- a $C_1$ to $C_{50}$ alkyl ester group by introducing a $C_1$ to $C_{50}$ monoalcohol during the synthesis,
- a $C_1$ to $C_{50}$ alkylamide group by taking as stopping group a monoacid if the silicone is $\alpha,\omega$-diaminated, or a monoamine if the silicone is an $\alpha,\omega$-dicarboxylic acid.

According to one embodiment of the invention, it is possible to use a copolymer of silicone polyamide and of hydrocarbon-based polyamide, i.e. a copolymer comprising moieties of formula (III) or (IV) and hydrocarbon-based polyamide moieties. In this case, the polyamide-silicone moieties may be arranged at the ends of the hydrocarbon-based polyamide.

Polyamide-based polymers containing silicones may be produced by silylic amidation of polyamides based on fatty acid dimer. This approach involves the reaction of free acid sites existing on a polyamide as end sites, with organosiloxane-monoamines and/or organosiloxane-diamines (amidation reaction), or alternatively with oligosiloxane alcohols or oligosiloxane diols (esterification reaction). The esterification reaction requires the presence of acid catalysts, as is known in the art. It is desirable for the polyamide containing free acid sites, used for the amidation or esterification reaction, to have a relatively high number of acid end groups (for example polyamides with high acid numbers, for example from 15 to 20).

For the amidation of the free acid sites of the hydrocarbon-based polyamides, siloxane diamines with 1 to 300, more particularly 2 to 50 and for example, 2, 6, 9.5, 12, 13.5, 23 or 31 siloxane groups, may be used for the reaction with hydrocarbon-based polyamides based on fatty acid dimers. Siloxane diamines containing 13.5 siloxane groups are preferred, and the best results are obtained with the siloxane diamine containing 13.5 siloxane groups and polyamides containing high numbers of carboxylic acid end groups.

The reactions may be carried out in xylene to extract the water produced from the solution by azeotropic distillation, or at higher temperatures (about 180 to 200°C) without solvent. Typically, the efficacy of the amidation and the reaction rates decrease when the siloxane diamine is longer, that is to say when the number of siloxane groups is higher. Free amine sites may be blocked after the initial amidation reaction of the diaminosiloxanes by reacting them either with a siloxane acid, or with an organic acid such as benzoic acid.

For the esterification of the free acid sites on the polyamides, this may be performed in boiling xylene with about 1% by weight, relative to the total weight of the reagents, of para-toluenesulphonic acid as catalyst.

These reactions carried out on the carboxylic acid end groups of the polyamide lead to the incorporation of silicone moieties only at the ends of the polymer chain.

It is also possible to prepare a copolymer of polyamide-silicone, using a polyamide containing free amine groups, by amidation reaction with a siloxane containing an acid group.

It is also possible to prepare a gelling agent based on a copolymer between a hydrocarbon-based polyamide and a silicone polyamide, by transamidation of a polyamide having, for example, an ethylene-diamine constituent, with an oligosiloxane-$\alpha,\omega$-diamine, at high temperature (for example 200 to 300°C), to carry out a transamidation such that the ethylenediamine component of the original polyamide is replaced with the oligosiloxane diamine.

The copolymer of hydrocarbon-based polyamide and of polyamide-silicone may also be a grafted copolymer comprising a hydrocarbon-based polyamide backbone with pendent oligosiloxane groups.

This may be obtained, for example :

- by hydrosilylation of unsaturated bonds in polyamides based on fatty acid dimers;
- by silylation of the amide groups of a polyamide; or
- by silylation of unsaturated polyamides by means of an oxidation, that is to say by oxidizing the unsaturated groups into alcohols or diols, to form hydroxyl groups that are reacted with siloxane carboxylic acids or siloxane alcohols. The olefinic sites of the unsaturated polyamides may also be epoxidized and the epoxy groups may then be reacted with siloxane amines or siloxane alcohols.

The polyorganosiloxane containing polymers used in the composition of the invention are most preferably polymers of the polyorganosiloxane type such as those described in documents US 5,874,069, US 5,919,441, US 6,051,216 and US 5,981,680, the entire disclosures of which are hereby incorporated by reference.

According to another embodiment of the invention, the polyorganoxiloxane containing polymer is a homopolymer or a copolymer comprising urethane or urea groups.

As previously discussed, the polymer may comprise polyorganosiloxane moieties containing two or more urethane and/or urea groups, either in the backbone of the polymer or on side chains or as pendent groups.

The polymers comprising at least two urethane and/or urea groups in the backbone may be polymers comprising at least one moiety corresponding to the following formula:

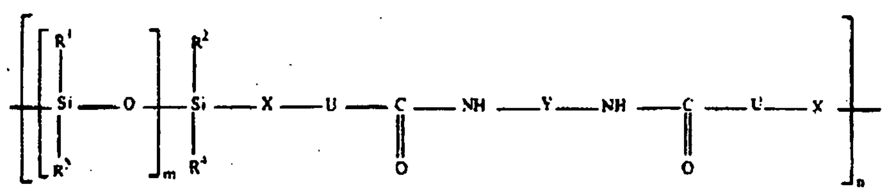

(VIII)

in which $R^1$, $R^2$, $R^3$, $R^4$, X, Y, m and n have the meanings given above for formula (I), and U represents -O- or -NH-, such that:

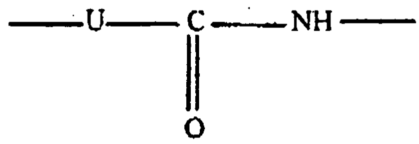

corresponds to a urethane or urea group.

In this formula (VIII), Y may be a linear or branched $C_1$ to $C_{40}$ alkylene group, optionally substituted with a $C_1$ to $C_{15}$ alkyl group or a $C_5$ to $C_{10}$ aryl group. Preferably, a $-(CH_2)_6-$ group is used.

Y may also represent a $C_5$ to $C_{12}$ cycloaliphatic or aromatic group that may be substituted with a $C_1$ to $C_{15}$ alkyl group or a $C_5$ to $C_{10}$ aryl group, for example a radical chosen from the methylene-4,4-biscyclohexyl radical, the radical derived from isophorone diisocyanate, 2,4- and 2,6-tolylenes, 1,5-naphthylene, p-phenylene and 4,4'-biphenylenemethane. Generally, it is preferred for Y to represent a linear or branched $C_1$ to $C_{40}$ alkylene radical or a $C_4$ to $C_{12}$ cycloalkylene radical.

Y may also represent a polyurethane or polyurea block corresponding to the condensation of several diisocyanate molecules with one or more molecules of coupling agents of the diol or diamine type. In this case, Y comprises several urethane or urea groups in the alkylene chain.

It may correspond to the formula :

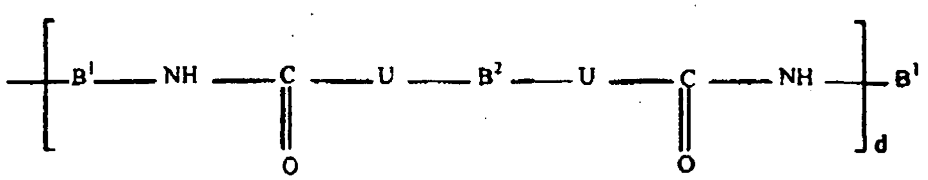

(IX)

in which $B^1$ is a group chosen from the groups given above for Y, U is -O- or -NH- and $B^2$ is chosen from:

- linear or branched $C_1$ to $C_{40}$ alkylene groups, which can optionally bear an ionizable group such as a carboxylic acid or sulphonic acid group, or a neutralizable or quaternizable tertiary amine group,
- $C_5$ to $C_{12}$ cycloalkylene groups, optionally bearing alkyl substituents, for example one to three methyl or ethyl groups, or alkylene, for example the diol radical: cyclohexanedimethanol,
- phenylene groups that may optionally bear $C_1$ to $C_3$ alkyl substituents, and
- groups of formula:

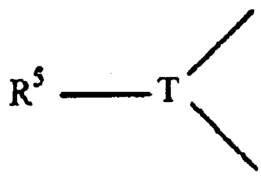

in which T is a hydrocarbon-based trivalent radical possibly containing one or more hetero atoms such as oxygen, sulphur and nitrogen and $R^5$ is a polyorganosiloxane chain or a linear or branched $C_1$ to $C_{50}$ alkyl chain.

T can represent, for example :

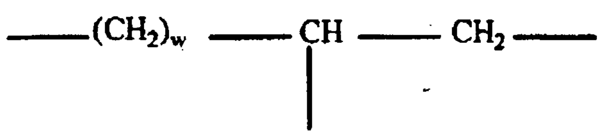

or

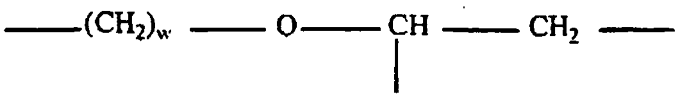

with w being an integer ranging from 1 to 10 and $R^5$ being a polyorganosiloxane chain.

When Y is a linear or branched $C_1$ to $C_{40}$ alkylene group, the $-(CH_2)_2-$ and $-(CH_2)_6-$ groups are preferred.

In the formula given above for Y, d may be an integer ranging from 0 to 5, preferably from 0 to 3 and more preferably equal to 1 or 2.

Preferably, B2 is a linear or branched C1 to C40 alkylene group, in particular $-(CH_2)_2-$ or $-(CH_2)_6-$ or a group:

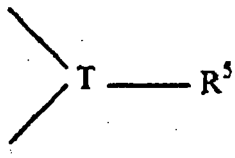

with $R^5$ being a polyorganosiloxane chain.

As previously discussed, the polyorganosiloxane containing polymer may be formed from silicone urethane and/or silicone urea moieties of different length and/or constitution, and may be in the form of block or random copolymers.

According to the invention, the silicone may also comprise urethane and/or urea groups no longer in the backbone but as side branches.

In this case, the polymer may comprise at least one moiety of formula:

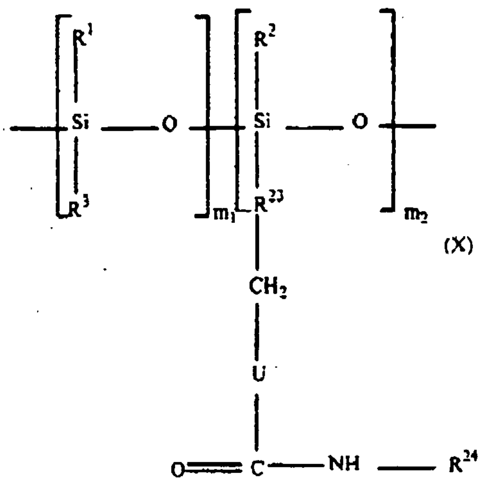

(X)

in which $R^1$, $R^2$, $R^3$, $m_1$ and $m_2$ have the meanings given above for formula (I),

- U represents O or NH,
- $R^{23}$ represents a $C_1$ to $C_{40}$ alkylene group, optionally comprising one or more hetero atoms chosen from O and N, or a phenylene group, and
- $R^{24}$ is chosen from linear, branched or cyclic, saturated or unsaturated $C_1$ to $C_{50}$ alkyl groups, and phenyl groups optionally substituted with one to three $C_1$ to $C_3$ alkyl groups.

  The polymers comprising at least one moiety of formula (X) contain siloxane units and urea or urethane groups, and they may be used, for example, as gelling agents in the compositions of the invention.

The siloxane polymers may have a single urea or urethane group by branching or may have branches containing two urea or urethane groups, or alternatively they may contain a mixture of branches containing one urea or urethane group and branches containing two urea or urethane groups.

They may be obtained from branched polysiloxanes, comprising one or two amino groups by branching, by reacting these polysiloxanes with monoisocyanates.

As examples of starting polymers of this type containing amino and diamino branches, mention may be made of the polymers corresponding to the following formulae:

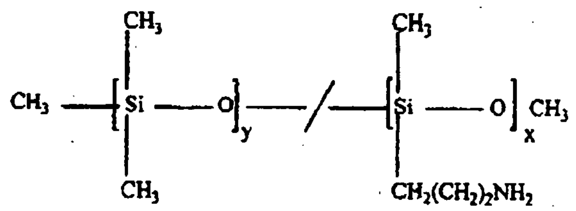

y=57 ; x = 3

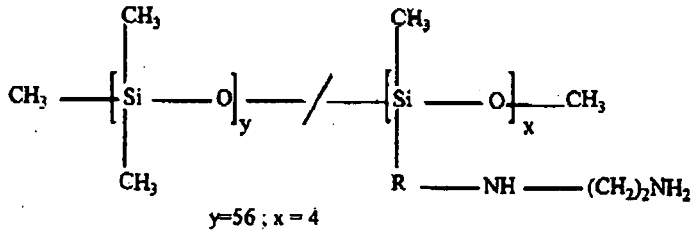

y=56 ; x = 4

In these formulae, the symbol "/" indicates that the segments may be of different lengths and in a random order, and R represents a linear aliphatic group preferably containing 1 to 6 carbon atoms, including 1 to 3 carbon atoms.

Such polymers containing branching may be formed by reacting a siloxane polymer, containing at least three amino groups per polymer molecule, with a compound containing only one monofunctional group (for example an acid, an isocyanate or an isothiocyanate) to react this monofunctional group with one of the amino groups and to form groups capable of establishing hydrogen interactions. The amino groups may be on side chains extending from the main chain of the siloxane polymer, such that the groups capable of establishing hydrogen interactions are formed on these side chains, or alternatively the amino groups may be at the ends of the main chain, such that the groups capable of hydrogen interaction will be end groups of the polymer.

As a procedure for forming a polymer containing siloxane units and groups capable of establishing hydrogen interactions, mention may be made of the reaction of a siloxane diamine and of a diisocyanate in a silicone solvent so as to provide a gel directly. The reaction may be performed in a silicone fluid, the resulting product being dissolved in the silicone fluid, at high temperature, the temperature of the system then being reduced to form the gel.

The polymers that are preferred for incorporation into the compositions according to the present invention are siloxane-urea copolymers that are linear and that contain urea groups as groups capable of establishing hydrogen interactions in the backbone of the polymer.

As an illustration of a polysiloxane ending with four urea groups, mention may be made of the polymer of formula:

(Ph=Phenyl)

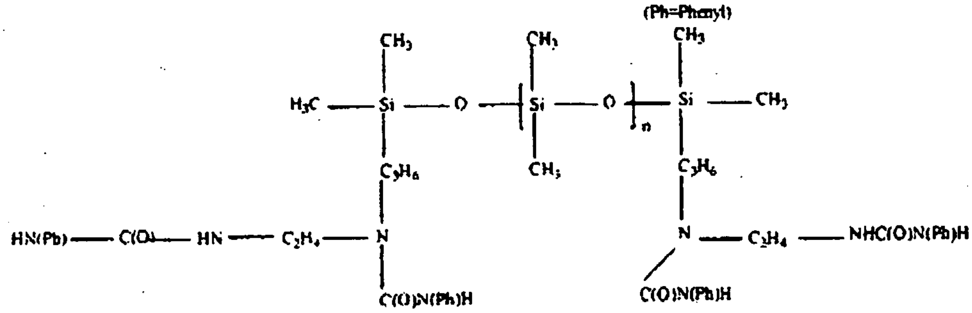

(XI)

in which Ph is a phenyl group and n is a number larger than 0, which includes, at least 1, 2 to 500, 2 to 200, from 1 to 300, in particular from 1 to 100, and all values and subranges there between, for example 50.

This polymer is obtained by reacting the following polysiloxane containing amino groups :

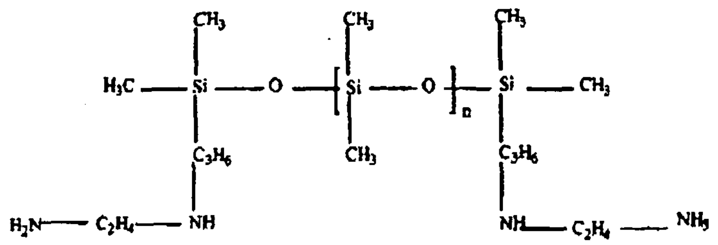

(n-50)

with phenyl isocyanate.

The polymers of formula (VIII) comprising urea or urethane groups in the chain of the silicone polymer may be obtained by reaction between a silicone containing $\alpha,\omega$-$NH_2$ or -OH end groups, of formula :

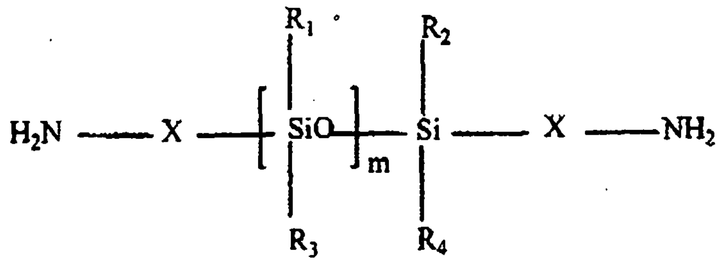

in which m, $R^1$, $R^2$, $R^3$, $R^4$ and X are as defined for formula (I) and a diisocyanate OCN-Y-NCO in which Y has the meaning given in formula (I); and optionally a diol or diamine coupling agent of formula $H_2N$-$B^2$-$NH_2$ or HO-$B^2$-OH, in which $B^2$ is as defined in formula (IX).

According to the stoichiometric proportions between the two reagents, diisocyanate and coupling agent, Y may have the formula (IX) with d equal to 0 or d equal to 1 to 5.

As in the case of the polyamide silicones of formula (II) or (III), it is possible to use in the invention polyurethane or polyurea silicones containing moieties of different length and structure, in particular moieties whose lengths differ by the number of silicone units. In this case, the copolymer may correspond, for example, to the formula :

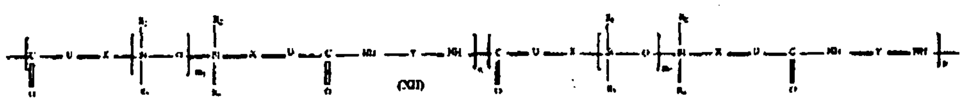

in which $R^1$, $R^2$, $R^3$, $R^4$, X, Y and U are as defined for formula (VIII) and $m_1$, $m_2$, n and p are as defined for formula (V).

Branched polyurethane or polyurea silicones may also be obtained using, instead of the diisocyanate OCN-Y-NCO, a triisocyanate of formula:

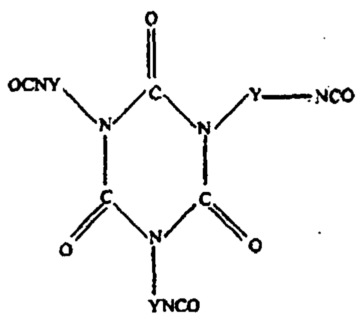

A polyurethane or polyurea silicone containing branches comprising an organosiloxane chain with groups capable of establishing hydrogen interactions is thus obtained. Such a polymer comprises, for example, a moiety corresponding to the formula ;

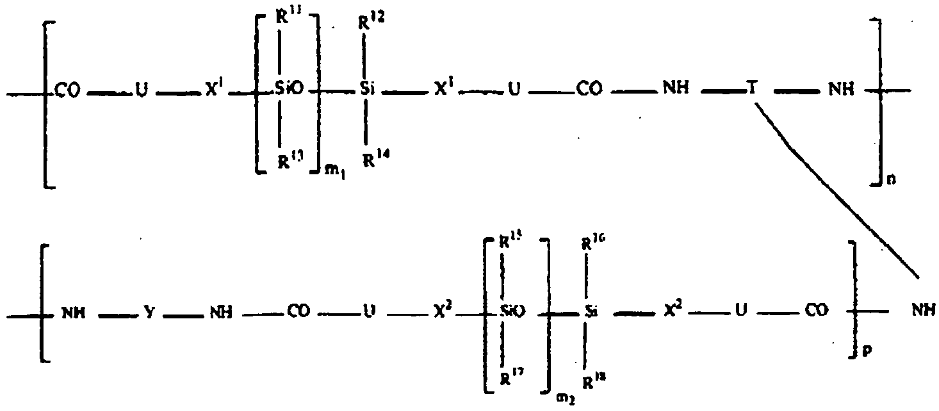

in which $X^1$ and $X^2$, which are identical or different, have the meaning given for X in formula (I), n is as defined in formula (I), Y and T are as defined in formula (I), $R^{11}$ to $R^{18}$ are groups chosen from the same group as $R^1$ to $R^4$, $m_1$ and $m_2$ are as defined above.

As in the case of the polyamides, this copolymer can also comprise polyurethane silicone moieties without branching.

In another embodiment of the invention, the siloxane-based polyureas and polyurethanes that are preferred are:

- polymers of formula (VIII) in which m is from 15 to 300, for example, 15 to 100 and all values and subranges there between;
- mixtures of two or more polymers in which at least one polymer has a value of m in the range from 15 to 50 and at least one polymer has a value of m in the range from 30 to 300, including all values and subranges there between;
- polymers of formula (XII) with $m_1$ chosen in the range from 15 to 50 and $m_2$ chosen in the range from 30 to 500 with the portion corresponding to $m_1$ representing 1% to 99% by weight of the total weight of the polymer and the portion corresponding to $m_2$ representing 1% to 99% by weight of the total weight of the polymer;
- mixtures of polymer of formula (VIII) combining

  1) 80% to 99% by weight of a polymer in which n is equal to 2 to 10 and in particular 3 to 6, and
  2) 1% to 20% of a polymer in which n is in the range from 5 to 500 and in particular from 6 to 100,

- copolymers comprising two moieties of formula (VIII) in which at least one of the groups Y contains at least one hydroxyl substituent;
- polymers of formula (VIII) synthesized with at least one portion of an activated diacid (diacid chloride, dianhydride or diester) instead of the diacid;
- polymers of formula (VIII) in which X represents $-(CH_2)_3-$ or $-(CH_2)_{10}-$; and
- polymers of formula (VIII) in which the polymers end with a multifunctional chain chosen from the group consisting of monofunctional amines, monofunctional acids, monofunctional alcohols, including fatty acids, fatty alcohols and fatty amines, such as, for example, octylamine, octanol, stearic acid and stearyl alcohol.

As in the case of the polyamides, copolymers of polyurethane or polyurea silicone and of hydrocarbon-based polyurethane or polyurea may be used in the invention by performing the reaction for synthesizing the polymer in the presence of an α, ω-difunctional block of non-silicone nature, for example a polyester, a polyether or a polyolefin.

As has been seen previously, homopolymers or copolymers of the invention may contain siloxane moieties in the main chain of the polymer and groups capable of establishing hydrogen interactions, either in the main chain of the polymer or at the ends thereof, or on side chains or branches of the main chain. This may correspond to the following five arrangements :

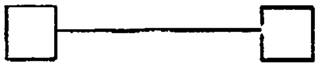
(1)

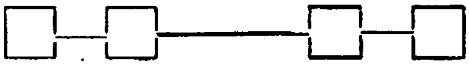
(2)

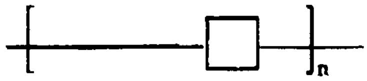
(3)

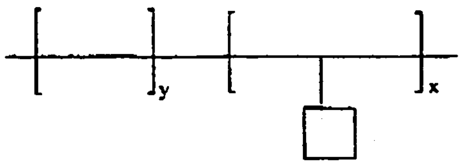
(4)

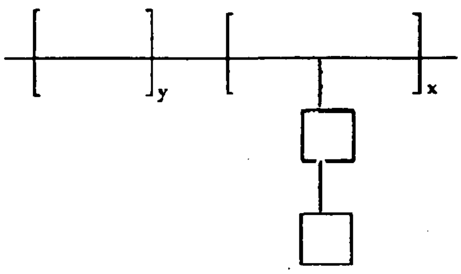
5

in which the continuous line is the main chain of the siloxane polymer and the squares represent the groups capable of establishing hydrogen interactions.

In case (1), the groups capable of establishing hydrogen interactions are arranged at the ends of the main chain.

In case (2), two groups capable of establishing hydrogen interactions are arranged at each of the ends of the main chain.

In case (3), the groups capable of establishing hydrogen interactions are arranged within the main chain in repeating moieties.

In cases (4) and (5), these are copolymers in which the groups capable of establishing hydrogen interactions are arranged on branches of the main chain of a first series of moieties that are copolymerized with moieties not comprising groups capable of establishing hydrogen interactions. Preferably, the values n, x and y are such that the polymer has the desired properties in terms of an agent for gelling fatty phases, preferably fatty phases based on silicone oil.

As examples of polymers that may be used, mention may be made of the silicone polyamides obtained in accordance with the disclosure in US-A-5981680, the entire disclosure of which is hereby incorporated by reference.

Further examples of polyorganosiloxane containing polymers are set forth in U.S. patents 6,503,632 and 6,569,955, both of which are hereby incorporated by reference in their entirety.

As noted above, the polymers of the present invention can be solid or liquid at room temperature. When solid, the polymers preferably have a softening point from 50 to 130°C. Most preferably, they have a softening point ranging from 65 to 150°C, including from 70°C to 130°C. This softening point is lower than that of other structuring polymers, which facilitates the use of the polymers that are the subject of the invention, and limits the deteriorations of the liquid fatty phase.

As noted above, the polyorganosiloxane containing polymers of the present invention contain both siloxane units and at least two groups capable of establishing hydrogen interactions such as amide linkages. The siloxane units can provide compatibility with a silicone fluid, if present, (for example with the cyclomethicones), while the groups capable of establishing hydrogen interactions and the spacing and selection of the locations of the amide linkages can facilitate gelation and the formation of cosmetic products.

In one embodiment, the polyorganosiloxane containing polymer of the present invention is present in an amount

effective to provide transfer resistant properties, and may also provide at least one of the following properties: pliability, softness, and wearing comfort. In addition, it is preferred that the compositions of the invention exhibit flexibility and/or good adherence on the keratinous substance to which the compositions have been applied. In another preferred embodiment, the compositions of the present invention when applied to the keratinous substance are substantially non-tacky.

In the composition of the present invention, the polyorganosiloxane-containing polymers are preferably present in an amount of 0.1-80 percent by weight, more preferably from 0.5 to 30 percent by weight and most preferably from 1 to 20 percent by weight of the total weight of the composition.

Depending on the intended application, such as a stick, hardness of the composition may also be considered. The hardness of a composition may, for example, be expressed in gramforce (gf). The composition of the present invention may, for example, have a hardness ranging from 20 gf to 2000 gf, such as from 20 gf to 900 gf, and further such as from 20 gf to 600 gf.

This hardness is measured in one of two ways. A first test for hardness is according to a method of penetrating a probe into the composition and in particular using a texture analyzer (for example TA-XT2i from Rheo) equipped with an ebonite cylinder of height 25 mm and diameter 8 mm. The hardness measurement is carried out at 20°C at the center of 5 samples of the composition. The cylinder is introduced into each sample of composition at a pre-speed of 2 mm/s and then at a speed of 0.5 mm/s and finally at a post-speed of 2 mm/s, the total displacement being 1 mm. The recorded hardness value is that of the maximum peak observed. The measurement error is ± 50gf.

The second test for hardness is the "cheese wire" method, which involves cutting an 8.1 mm or preferably 12.7 mm in diameter stick composition and measuring its hardness at 20°C using a DFGHS 2 tensile testing machine from Indelco-Chatillon Co. at a speed of 100 mm/minute. The hardness value from this method is expressed in grams as the shear force required to cut a stick under the above conditions. According to this method, the hardness of compositions according to the present invention which may be in stick form may, for example, range from 30 gf to 300 gf, such as from 30 gf to 250 gf, for a sample of 8.1 mm in diameter stick, and further such as from 30 gf to 200 gf, and also further such as from 30 gf to 120 gf for a sample of 12.7 mm in diameter stick.

The hardness of the composition of the present invention may be such that the compositions are self-supporting and can easily disintegrate to form a satisfactory deposit on a keratinous material. In addition, this hardness may impart good impact strength to the inventive compositions, which may be molded or cast, for example, in stick or dish form.

The skilled artisan may choose to evaluate a composition using at least one of the tests for hardness outlined above based on the application envisaged and the hardness desired. If one obtains an acceptable hardness value, in view of the intended application, from at least one of these hardness tests, the composition falls within preferred embodiments of the invention.

As is evident, the hardness of the composition according to preferred embodiments of the invention may, for example, be such that the composition is advantageously self-supporting and can disintegrate easily to form a satisfactory deposit on the skin and/or the lips and/or superficial body growths, such as keratinous fibers. In addition, with this hardness, the composition of the invention may have good impact strength.

According to preferred embodiments of the present invention, the composition in stick form may have the behavior of a deformable, flexible elastic solid, giving noteworthy elastic softness on application. The compositions in stick form of the prior art do not have these properties of elasticity and flexibility.

LIQUID FATTY PHASE

According to preferred embodiments of the present invention, cosmetic compositions comprising at least one polyorganosiloxane containing polymer and a liquid fatty phase are provided, Preferably, the liquid fatty phase comprises at least one volatile oil, e.g., a silicone volatile oil, a hydrocarbon volatile oil, or a mixture thereof.

In accordance with this embodiment, the liquid fatty phase may contain, independently or in combinations, volatile silicone oils, non-volatile silicone oils, volatile non-silicone oils and non-volatile non-silicone oils. In one embodiment, the compositions of the present invention are substantially free of silicone oils (i.e., contain less than about 0.1% silicone oils). In another embodiment, the compositions are substantially free of non-silicone oils (i.e., contain less than about 0.1% non-silicone oils). In another embodiment, the compositions are substantially free of non-volatile oils (i.e., contain less than about 0.1% non-volatile oils).

According to the invention, when volatile oils are present, these volatile oils permit an easier application of the composition on the skin, lips or keratinous fibers.

According to one embodiment, the composition may contain one or more volatile silicone oils. Examples of such volatile silicone oils include linear or cyclic silicone oils having a viscosity at room temperature less than or equal to 6 cSt and having from 2 to 7 silicon atoms, these silicones being optionally substituted with alkyl or alkoxy groups of 1 to 10 carbon atoms. Specific oils that may be used in the invention include octamethyltetrasiloxane, decamethylcyclopentasiloxane, dodecamethylcyclohexasiloxane, heptamethyloctyltrisiloxane, hexamethyldisiloxane, decamethyltetrasiloxane, dodecamethylpentasiloxane and their mixtures. Other volatile oils which may be used include KF 96A of 6 cSt viscosity, a commercial product from Shin Etsu having a flash point of 94°C. Preferably, the volatile silicone oils have a flash point of at least 40°C.

Non-limiting examples of volatile silicone oils are listed in Table 1 below.

Table 1

| Compound | Flash Point (°C) | Viscosity (cSt) |
|---|---|---|
| Octyltrimethicone | 93 | 1.2 |
| Hexyltrimethicone | 79 | 1.2 |
| Decamethylcyclopentasiloxane (cyclopentasiloxane or D5) | 72 | 4.2 |
| Octamethylcyclotetrasiloxane (cyclotetradimethylsiloxane or D4) | 55 | 2.5 |
| Dodecamethylcxclohexasiloxane (D6) | 93 | 7 |
| Decamethyltetrasiloxane (L4) | 63 | 1.7 |
| KF-96 A from Shin Etsu | 94 | 6 |
| PDMS (polydimethylsiloxane) DC 200 (1.5cSt) from Dow Corning | 56 | 1.5 |
| PDMS DC 200 (2cSt) from Dow Corning | 87 | 2 |
| PDMS DC 200 (5cSt) from Dow Corning | 134 | 5 |
| PDMS DC 200 (3St) from Dow Corning | 102 | 3 |

Examples of other silicone oils that may be used in the invention include non-volatile linear polydimethylsiloxanes (PDMSs), that are liquid at room temperature; polydimethylsiloxanes comprising alkyl, alkoxy or phenyl groups, which are pendent and/or at the end of a silicone chain, these groups each containing from 2 to 24 carbon atoms; phenylsilicones, for instance phenyl trimethicones, phenyl dimethicones, phenyl trimethylsiloxydiphenylsiloxanes, diphenyl dimethicones, diphenyl methyldiphenyl trisiloxanes and 2-phenylethyl trimethylsiloxysilicates.

Further, a volatile linear silicone oil may be employed in the compositions of the present invention. Suitable volatile linear silicone oils include those described in U.S. patent no. 6,338,839 and WO03/042221, the contents of which are incorporated herein by reference. In one embodiment the volatile linear silicone oil is decamethyltetrasiloxane. In another embodiment, the decamethyltetrasiloxane is further combined with another solvent that is more volatile than decamethyltetrasiloxane.

The volatility of the solvents/oils can be determined using the evaporation speed as set forth in U.S. patent no. 6,338,839.

According to other preferred embodiments, the composition may contain one or more non-silicone volatile oils and may be selected from volatile hydrocarbon oils, alcohols, volatile esters and volatile ethers. Examples of such volatile non-silicone oils include, but are not limited to, volatile hydrocarbon oils having from 8 to 16 carbon atoms and their mixtures and in particular branched $C_8$ to $C_{16}$ alkanes such as $C_8$ to $C_{16}$ isoalkanes (also known as isoparaffins), isododecane, isodecane, isohexadecane, and for example, the oils sold under the trade names of Isopar or Permethyl, the $C_8$ to $C_{16}$ branched esters such as isohexyl or isodecyl neopentanoate and their mixtures. Preferably, the volatile non-silicone oils have a flash point of at least 40°C.

Non-limiting examples of volatile non-silicone volatile oils are given in Table 2 below.

Table 2

| Compound | Flash Point (°C) |
|---|---|
| Isododecane | 43 |
| Isohexadecane | 102 |
| Isodecyl Neopentanoate | 118 |
| Propylene glycol n-butyl ether | 60 |
| Ethyl 3-ethoxypropionate | 58 |
| Propylene glycol methylether acetate | 46 |
| Isopar L (isoparaffin $C_{11}$-$C_{13}$) | 62 |
| Isopar H (isoparaffin $C_{11}$-$C_{12}$) | 56 |

Examples of other non-silicone oils which can be used in the compositions of the present invention include polar oils such as:

- hydrocarbon-based plant oils with a high triglyceride content consisting of fatty acid esters of glycerol, the fatty acids of which may have varied chain lengths, these chains possibly being linear or branched, and saturated or unsaturated; these oils are especially wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, for instance those sold by the company Stearineries Dubois or those sold under the names Miglyol 810, 812 and 818 by the company Dynamit Nobel;
- synthetic oils or esters of formula $R_5COOR_6$ in which $R_5$ represents a linear or branched higher fatty acid residue containing from 1 to 40 carbon atoms, including from 7 to 19 carbon atoms, and $R_6$ represents a branched hydrocarbon-based chain containing from 1 to 40 carbon atoms, including from 3 to 20 carbon atoms, with $R_6 + R_7 \geq 10$, such as, for example, Purcellin oil (cetostearyl octanoate), isononyl isononanoate, $C_{12}$ to $C_{15}$ alkyl benzoate, isopropyl myristate, 2-ethylhexyl palmitate, and octanoates, decanoates or ricinoleates of alcohols or of polyalcohols ; hydroxylated esters, for instance isostearyl lactate or diisostearyl malate; and pentaerythritol esters;
- synthetic ethers containing from 10 to 40 carbon atoms;
- $C_8$ to $C_{26}$ fatty alcohols, for instance oleyl alcohol; and
- mixtures thereof.

Preferably, the liquid fatty phase, when present, represents from 5% to 98.4% of the total weight of the composition, more preferably from 10% to 80% of the total weight of the composition, and most preferably from 20% to 75%.
FILM FORMERS
The composition of the present invention advantageously also includes one or more film forming agents. Film forming agents are known in the art.
According to preferred embodiments of the present invention, compositions comprising at least one polyorganosiloxane containing polymer and at least one silicone film forming agent, preferably an MK or MQ resin or mixtures thereof, are provided.
Silicone resin nomenclature is known in the art as "MDTQ" nomenclature, whereby a silicone resin is described according to the various monomeric siloxane units which make up the polymer.
Each letter of "MDTQ" denotes a different type of unit. The letter M denotes the monofunctional unit $(CH_3)_3SiO_{1/2}$. This unit is considered to be monofunctional because the silicone atom only shares one oxygen when the unit is part of a polymer. The "M" unit can be represented by the following structure:

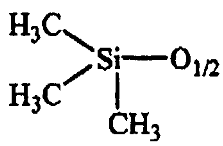

At least one of the methyl groups of the M unit may be replaced by another group, e.g., to give a unit with formula $[R(CH_3)_2]SiO_{1/2}$, as represented in the following structure:

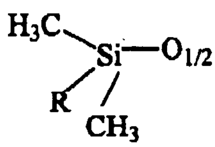

wherein R is chosen from groups other than methyl groups. Non-limiting examples of such groups other than methyl groups include alkyl groups other than methyl groups, alkene groups, alkyne groups, hydroxyl groups, thiol groups, ester groups, acid groups, ether groups, wherein the groups other than methyl groups may be further substituted.
The symbol D denotes the difunctional unit $(CH_3)_2SiO_{2/2}$ wherein two oxygen atoms bonded to the silicone atom are used for binding to the rest of the polymer. The "D" unit, which is the major building block of dimethicone oils, can be represented as:

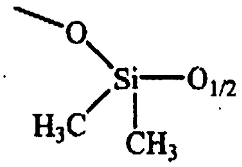

At least one of the methyl groups of the D unit may be replaced by another group, e.g., to give a unit with formula $[R(CH_3)_2]SiO_{1/2}$.

The symbol T denotes the trifunctional unit, $(CH_3)SiO_{3/2}$ and can be represented as:

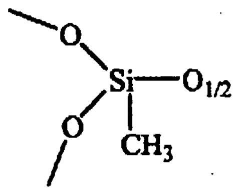

At least one of the methyl groups of the T unit may be replaced by another group, e.g., to give a unit with formula $[R(CH_3)_2]SiO_{1/2}$.

Similarly, the symbol Q denotes the tetrafunctional unit, $SiO_{4/2}$ wherein all four oxygens bonded to the silicone atom are bonded to the rest of the polymer.

Thus, a vast number of different silicone polymers can be manufactured. Further, it would be clear to one skilled in the art that the properties of each of the potential silicone polymers will vary depending on the type(s) of monomer (s), the type(s) of substitution(s), the size of the polymeric chain, the degree of cross linking, and size of any side chain(s).

Non-limiting examples of silicone polymers include silanes, siloxanes, siloxysilicates, and silsesquioxanes. A non-limiting example of such a siloxane is polydimethylsiloxane (PDMS). Polydimethylsiloxanes are generally composed of long straight chains of $(CH_3)_2SiO_{2/2}$ (i.e., D units) and have viscosities which are dependent on both the size of the polymer and the presence and nature of any substituent(s) on the polymer. A non-limiting example of a siloxysilicate is trimethylsiloxysilicate, which may be represented by the following formula:

$$[(CH_3)_3\text{-}Si\text{-}O]_x\text{-}(SiO_{4/2})_y$$

(i.e, MQ units) wherein x and y may, for example, range from 50 to 80. Silsesquioxanes, on the other hand, may be represented by the following formula:

$$(CH_3SiO_{3/2})_x$$

(i.e., T Units) wherein x may, for example, have a value of up to several thousand.

Polymethylsilsesquioxanes are silsesquioxanes that do not have a substituent replacing the methyl groups. Certain polymethylsilsesquioxanes have previously been used in hair care compositions. See, e.g., U.S. Pat. No. 5,246,694, the disclosure of which is incorporated herein by reference, which discloses a shampoo composition comprising a surfactant, an aqueous emulsion of highly viscous silicone in volatile silicone and a cationic polymer which is a derivative of guar gum. The highly viscous silicone disclosed therein may be chosen from silicone resins including a polymethylsilsesquioxane such as Resin MK (also called SiliconHarz MK) which is available from Wacker, and a siloxysilicate such as Resin MQ which is available from General Electric and Dow Corning.

The Resin MK and Resin MQ silicone resins may form a film after a volatile carrier has evaporated. The MQ film is generally hard and brittle at room temperature, while the MK film is generally continuous and flexible, i.e., not brittle. Depending on the application, plasticizers may be added to help obtain a more flexible, thus more comfortable, film.

In one embodiment, the silicone film former may be a polymethylsilsesquioxane film former such as Belsil PMS MK, also referred to as Resin MK, available from Wacker Chemie. This polymethylsilsesquioxane film former is a polymer comprising polymerized repeating units of $CH_3SiO_{3/2}$ (T units) and may also contain up to 1% by weight or by mole of units of the formula $(CH_3)_2SiO_{2/2}$ (D units). The weight-average molecular weight of this polymer has been estimated to be 10,000. It is believed that the polymers are in a "cage" and "ladder" configuration, as exemplified in the figures

below. The majority of the polymer is in the "ladder" configuration, wherein the ends of the polymer are capped with ethoxy ($CH_3CH_2O$) groups. The ethoxy groups are generally present in an amount of 4.5% by weight and the mole percent is generally 7% (silicone units). As ethoxy groups may react with water, a small and variable amount of SiOH may also be present in the polymer.

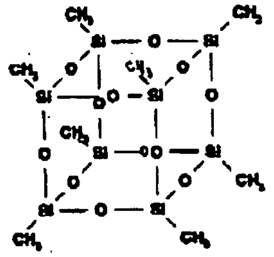

Cage

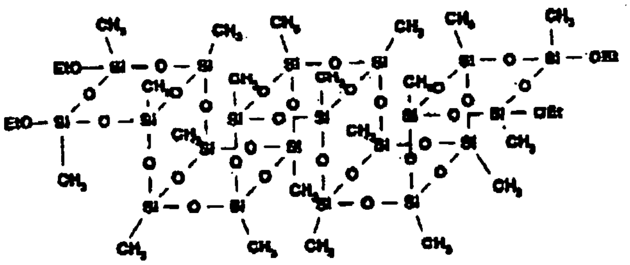

Ladder

Another non-limiting example of the at least one polymethylsilsesquioxane film former suitable for use in the present invention is KR-220L, which is available from SHIN-ETSU. This polymethylsilsesquioxane film former is composed of silicone T-units (i.e., those of formula $CH_3SiO_{3/2}$) and has Si-OH (or silanol) end units. There are no D units in KR-220L.

Other non-limiting examples of the at least one polymethylsilsesquioxane film former that may be useful in the practice of the invention include KR-242A (which is comprised of methyl T units (98%) and dimethyl D units (2%) and has Si-OH end units) and KR-251 (which is comprised of methyl T units (88%) and dimethyl D units (12%) and has Si-OH end units), both of which are available from SHIN-ETSU.

Depending on the application, the concentration of the at least one polymethylsilsesquioxane film former in the presently claimed composition may vary considerably. One of skill in the art will be able to determine routinely the amount of the at least one polymethylsilsesquioxane film former depending on the desired application.

In another embodiment, the silicone film former may be chosen from siloxysilicates. Preferably, the siloxysilicate is trimethylsiloxysilicate, which may or may not be in powder form. Trimethylsiloxysilicate (TMS) is commercially available from General Electric under the tradename SR1000 and from Wacker under the tradename TMS 803. TMS is also commercially available from Dow Chemical in a solvent, such as for example, cyclomethicone. However, according to the present invention, TMS may be used in the form of 100% active material, that is, not in a solvent.

Further non-limiting examples of the silicone film formers include silicone/(meth)acrylate copolymers, such as those as described in US Patent Nos. 5,061,481, 5,219,560, and 5,262,087, the disclosures of which are hereby incorporated by reference. Still further non-limiting examples of silicone film formers are non-polar silicone copolymers comprising repeating units of at least one polar (meth)acrylate unit and vinyl copolymers grafted with at least one non-polar silicone chain. Non-limiting examples of such copolymers are acrylates/dimethicone copolymers such as those commercially available from Shin-Etsu, for example, the product sold under the tradename KP-545, or acrylates/stearyl acrylate/dimethicone acrylates copolymers, such as those commercially available from Shin-Etsu, for example, the product sold under the tradename KP-561, and acrylates/behenyl acrylate/dimethicone acrylates copolymer, such as those commercially available from Shin-Etsu, for example, the product sold under the tradename KP-562.

Other non-limiting examples of silicone film formers suitable for use in the present invention are silicone esters comprising units of formulae (XIV) and (XV), disclosed in U.S. Pat. Nos. 6,045,782, 5,334,737, and 4,725,658, the disclosures of which are hereby incorporated by reference:

$$R_a R^E_b SiO_{[4-(a+b)/2]} \quad (XIV);$$

and

$$R'_x R^E_y SiO_{1/2} \quad (XV)$$

wherein

R and R', which may be identical or different, are each chosen from optionally substituted hydrocarbon groups;
a and b, which may be identical or different, are each a number ranging from 0 to 3, with the proviso that the sum of a and b is a number ranging from 1 to 3,
x and y, which may be identical or different, are each a number ranging from 0 to 3, with the proviso that the sum of x and y is a number ranging from 1 to 3;
$R^E$, which may be identical or different, are each chosen from groups comprising at least one carboxylic ester.

In one embodiment, RE groups are chosen from groups comprising at least one ester group formed from the reaction of at least one acid and at least one alcohol. In another embodiment, the at least one acid comprises at least two carbon atoms. In another embodiment, the at least one alcohol comprises at least ten carbon atoms. Non-limiting examples of the at least one acid include branched acids such as isostearic acid, and linear acids such as behenic acid. Non-limiting examples of the at least one alcohol include monohydric alcohols and polyhydric alcohols, such as n-propanol and branched etheralkanols such as (3,3,3-trimethylolpropoxy)propane.
Further non-limiting examples of the at least one silicone film former include liquid siloxy silicates and silicone esters such as those disclosed in U.S. Pat. No. 5,334,737, the disclosure of which is hereby incorporated by reference, such as diisostearoyl trimethylolpropane siloxysilicate and dilauroyl trimethylolpropane siloxy silicate, which are commercially available from General Electric under the tradenames SF 1318 and SF 1312, respectively.
Yet further non-limiting examples of the at least one silicone film former include polymers comprising a backbone chosen from vinyl polymers, methacrylic polymers, and acrylic polymers and at least one chain chosen from pendant siloxane groups and pendant fluorochemical groups. Non-limiting examples of such polymers comprise at least one unit derived from at least one A monomer, at least one unit derived from at least one C monomer, at least one unit derived from D monomers, and, optionally, at least one unit derived from at least one B monomer, wherein:
A, which may be identical or different, are each chosen from free-radically-polymerizable acrylic esters of at least one alcohol chosen from 1,1,-dihydroperfluoroalkanols, omega-hydridofluoroalkanols, fluoroalkylsulfonamido alcohols, cyclic fluoroalkyl alcohols, and fluoroether alcohols, and analogs of any of the foregoing at least one alcohols, and free-radically-polymerizable methacrylic esters of at least one alcohol chosen from 1,1,-dihydroperfluoroalkanols, omega-hydridofluoroalkanols, fluoroalkylsulfonamido alcohols, cyclic fluoroalkyl alcohols, and fluoroether alcohols, and analogs of any of the foregoing at least one alcohols;
B, which may be identical or different, are each chosen from reinforcing monomers which are copolymerizable with at least one A monomer;
C, which may be identical or different, are each chosen from monomers having the formula:
$$X(Y)_n Si(R)_{3-m} Z_m$$

wherein
X is chosen from vinyl groups which are copolymerizable with at least one A monomer and at least one B monomer,
**[0279]** Y is chosen from divalent allylene groups, divalent arylene groups, divalent alkarylene groups, and divalent aralkylene groups, wherein the groups comprise from 1 to 30 carbon atoms, and further wherein the groups optionally further comprise at least one group chosen from ester groups, amide groups, urethane groups, and urea groups;
n is zero or 1;
m is a number ranging from 1 to 3;
R, which may be identical or different, are each chosen from hydrogen, $C_1$- $C_4$ alkyl groups, aryl groups, and alkoxy groups; and
Z, which may be identical or different, are each chosen from monovalent siloxane polymeric groups; and D, which may be identical or different, are each chosen from free-radically-polymerizable acrylate copolymers and free-radically-polymerizable methacrylate copolymers. Such polymers and their manufacture are disclosed in U.S. Pat. Nos. 5,209,924 and 4,972,037, and WO 01/32737, the disclosures of which are hereby incorporated by reference.
Other non-limiting examples of the at least one silicone film former include silicone/acrylate graft terpolymers,

for example, those having the formula:

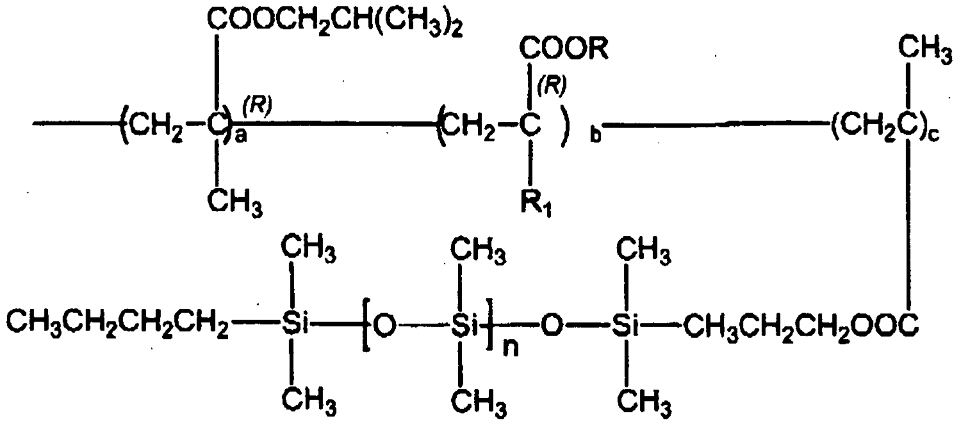

wherein

a, b, and c are present in a weight ratio of 69.9 : 0.1 : 30 respectively,

R and $R_1$, which may be identical or different, are each chosen from hydrogen and $C_1$-$C_6$ alkyl groups; and m is a number ranging from 100 - 150.

In an embodiment, m is chosen to provide a macromer having a molecular weight ranging from 8,000 to 12,000, such as 10,000. In another embodiment, m is a number ranging from 124-135, such as 130. Non-limiting examples of these copolymers are described in WO 01/32727 A1, the disclosure of which is hereby incorporated by reference.

Still other examples of suitable silicone film formers include copolymers comprising a backbone chosen from vinyl backbones, methacrylic backbones, and acrylic polymeric backbones and further comprising at least one pendant siloxane group. Non-limiting examples of such polymers are disclosed in U.S. Pat. Nos. 4,693,935, 4,981,903, 4,981,902, the disclosures of which are hereby incorporated by reference.

In an embodiment, the at least one copolymer comprises at least one A monomer, at least one C monomer, and, optionally at least one B monomer, wherein the at least one A monomer is chosen from free-radically-polymerizable vinyl monomers, free-radically-polymerizable methacrylate monomers, and free-radically-polymerizable acrylate monomers; the at least one B monomer, if present, is chosen from at least one reinforcing monomer copolymerizable with the at least one A monomer, and the at least one C monomer is chosen from monomers having the formula:

$$X(Y)_n\ Si(R)_{3-m}\ Zm$$

wherein:

X is chosen from vinyl groups which are copolymerizable with the at least one A monomer and with the at least one B monomer;
Y is chosen from divalent groups;
n is zero or 1;
m is a number ranging from 1 to 3;
R, which may be identical or different, are each chosen from hydrogen, optionally substituted $C_1$-$C_{10}$ alkyl groups, optionally substituted phenyl groups, and optionally substituted $C_1$-$C_{10}$ alkoxy groups; and
z, which may be identical or different, are each chosen from monovalent siloxane polymeric groups.

Non-limiting examples of A monomers include methacrylic acid esters of $C_1$-$C_{22}$ linear alcohols, methacrylic acid esters of $C_1$-$C_{12}$ of branched alcohols, styrene monomers, vinyl esters, vinyl chloride monomers, vinylidene chloride monomers, and acryloyl monomers.

Non-limiting examples of B monomers include acrylic monomers comprising at least one group chosen from hydroxyl, amino, and ionic groups, and methacrylic monomers comprising at least one group chosen from hydroxyl, amino, and ionic groups. Non-limiting examples of ionic groups include quaternary ammonium groups, carboxylate salts, and sulfonic acid salts.

The C monomers are the same as those described for the C monomers in the previous paragraphs.

Other non-limiting examples of the silicone film-former include a copolymer chosen from vinyl-silicone graft copolymers having the following formula and vinyl-silicone block copolymers having the following formula:

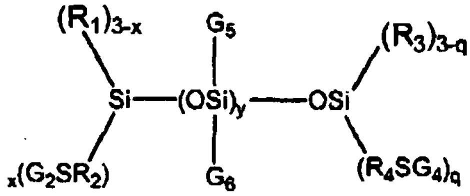

wherein

$G_5$, which may be identical or different, are each chosen from alkyl groups, aryl groups, aralkyl groups, alkoxy groups, alkylamino groups, fluoroalkyl groups, hydrogen, and -ZSA groups, wherein
A is chosen from vinyl polymeric segments comprising at least one polymerized free-radically-polymerizable monomer, and
Z is chosen from divalent $C_1$-$C_{10}$ alkylene groups, divalent aralkylene groups, divalent arylene groups, and divalent alkoxylalkylene groups. In an embodiment Z is chosen from methylene groups and propylene groups.
$G_6$, which may be identical or different, are each chosen from alkyl groups, aryl groups, aralkyl groups, alkoxy groups, alkylamino groups, fluoroalkyl groups, hydrogen, and -ZSA groups, as defined above;
$G_2$ comprises A;
$G_4$ comprises A;
$R_1$, which may be identical or different, are each chosen from alkyl groups, aryl groups, aralkyl groups, alkoxy groups, alkylamino groups, fluoroalkyl groups, hydrogen, and hydroxyl. In one embodiment, $R_1$ is chosen from $C_1$-$C_4$ alkyl groups, such as methyl groups, and hydroxyl.
$R_2$, which may be identical or different, are each chosen from divalent $C_{1-10}$ alkylene groups, divalent arylene groups, divalent aralkylene groups, and divalent alkoxyalkylene groups. In one embodiment, $R_2$ is chosen from divalent $C_1$-$C_3$ alkylene groups and divalent $C_7$-$C_{10}$ aralkylene groups. In another embodiment, $R_2$ is chosen from -$CH_2$-groups and divalent 1,3-propylene groups.
$R_3$, which may be identical or different, are each chosen from alkyl groups, aryl groups, aralkyl groups alkoxy groups, alkylamino groups, fluoroalkyl groups, hydrogen, and hydroxyl. In one embodiment, $R_3$ is chosen from $C_1$-$C_4$ alkyl groups and hydroxyl. In another embodiment, $R_3$ is chosen from methyl groups.
$R_4$, which may be identical or different, are each chosen from divalent $C_1$-$C_{10}$ alkylene groups, divalent arylene groups, divalent aralkylene groups, and divalent alkoxyalkylene groups. In one embodiment, $R_4$ is chosen from divalent $C_1$-$C_3$ alkylene groups and divalent $C_7$-$C_{10}$ aralkylene groups. In another embodiment, $R_4$ is chosen from divalent - $CH_2$ - groups and divalent 1,3-propylene groups.
x is a number ranging from 0 to 3;
y is a number greater than or equal to 5. In an embodiment, y ranges from 10 to 270, and in another embodiment, y ranges from 40 to 270.
q is a number ranging from 0 to 3;

Non-limiting examples of these polymers are described in U.S. Pat. No. 5,468,477, the disclosure of which is hereby incorporated by reference. A non-limiting example of such polymers is poly(dimethylsiloxane)-g-poly(isobutyl methacrylate), which is commercially available from 3M Company under the tradename VS 70 IBM.

According to preferred embodiments, the silicone film former is present in the composition in an amount ranging from 0.1% to 30% by weight relative to the total weight of the composition. Preferably, the silicone film former is present in an amount ranging from 0.5% to 20% by weight relative to the total weight of the composition, and more preferably from 1% to 10%. One of ordinary skill in the art will recognize that the silicone film former of the present invention may be commercially available, and may come from suppliers in the form of a dilute solution. The amounts of the silicone film former disclosed herein therefore reflect the weight percent of active material.

In a preferred embodiment, the polyorganosilxane polymer and the film forming agent are solid. The composition is prepared by heating the solids sufficient to combine and form compositions as described herein. This combination of solid polyorganosilxane polymer and film forming agent provide beneficial transfer-resistant, long-wear compositions.

According to preferred embodiments, cosmetic compositions comprising at least one polyorganosiloxane containing polymer and at least one coloring agent are provided. Preferably, such colored cosmetic compositions are lip compositions (for example, lipstick or liquid lip colors) or foundations.

According to this embodiment, the at least one coloring agent is preferably chosen from pigments, dyes, such as liposoluble dyes, nacreous pigments, and pearling agents.

Representative liposoluble dyes which may be used according to the present invention include Sudan Red,

DC Red 17, DC Green 6, β-carotene, soybean oil, Sudan Brown, DC Yellow 11, DC Violet 2, DC Orange 5, annatto, and quinoline yellow. The liposoluble dyes, when present, generally have a concentration ranging up to 20% by weight of the total weight of the composition, such as from 0.0001% to 6%.

The nacreous pigments which may be used according to the present invention may be chosen from white nacreous pigments such as mica coated with titanium or with bismuth oxychloride, colored nacreous pigments such as titanium mica with iron oxides, titanium mica with ferric blue or chromium oxide, titanium mica with an organic pigment chosen from those mentioned above, and nacreous pigments based on bismuth oxychloride. The nacreous pigments, if present, be present in the composition in a concentration ranging up to 50% by weight of the total weight of the composition, such as from 0.1% to 20%, preferably from 0.1% to 15%.

The pigments, which may be used according to the present invention, may be chosen from white, colored, inorganic, organic, polymeric, nonpolymeric, coated and uncoated pigments. Representative examples of mineral pigments include titanium dioxide, optionally surface-treated, zirconium oxide, zinc oxide, cerium oxide, iron oxides, chromium oxides, manganese violet, ultramarine blue, chromium hydrate, and ferric blue. Representative examples of organic pigments include carbon black, pigments of D & C type, and lakes based on cochineal carmine, barium, strontium, calcium, and aluminum.

The pigments may be present in the composition in a concentration ranging up to 50% by weight of the total weight of the composition, such as from 0.5% to 40%, and further such as from 2% to 30%. In the case of certain products, the pigments, including nacreous pigments, may, for example, represent up to 50% by weight of the composition.

According to preferred embodiments of the present invention, the compositions comprising at least one polyorganosiloxane containing polymer are anhydrous. By "anhydrous," it is meant that the composition contains substantially no water (that is, less than about 0.1% by weight of the composition of water).

According to other preferred embodiments, the compositions comprising at least one polyorganosiloxane containing polymer further comprise water. In this embodiment, water is preferably present in an amount ranging from about 0.1 to about 70 %, preferably from about 0.5 to 50%, and more preferably from about 1 to about 30 % relative to the total weight of the composition. Preferably, such water-containing cosmetic compositions are lip compositions (for example, lipstick or liquid lip colors), foundations or mascaras, and are emulsions or dispersions.

Additional ingredients which offer similar cosmetic properties as the short chain esters are short chain ethers which may be represented as J-O-K

where J and K are identical or different and represent a linear or branched alkyl radical from 1 to 40 carbon atoms, preferably from 7 to 19 carbon atoms, possibly including one or more double bonds. An example of such an ether includes dicapryl ether.

LIPOSOLUBLE OR DISPERSIBLE POLYMERS

The basecoat compositions of the invention also can contain at least one polymer that is liposoluble or dispersible in the medium, other than the polyorganosiloxane containing polymer, and may have film-forming properties and may have, for example, an average molecular weight of from 500 to 1,000,000, such as from 1,000 to 500,000, and for example, further such as from 5,000 to 100,000, and even further such as from 5,000 to 20,000. This at least one liposoluble polymer may contribute towards increasing the viscosity and/or improving the staying power of the film. The at least one liposoluble polymer can have a softening point of not more than 30°C.

As examples of liposoluble polymers which can be used in the invention, mention may be made of: polyalkylenes, in particular polybutene, poly(meth)acrylates, alkylcelluloses with a linear or branched, saturated or unsaturated $C_1$ to $C_8$ alkyl radical, such as ethylcellulose and propylcellulose, silicone polymers that are compatible with the fatty phase, as well as vinylpyrrolidone (VP) copolymers, and mixtures thereof.

Vinylpyrrolidone copolymers, copolymers of a $C_2$ to $C_{30}$, such as $C_3$ to $C_{22}$ alkene, and combinations thereof, can be used. As examples of VP copolymers which can be used in the invention, mention may be made of VP/vinyl acetate, VP/ethyl methacrylate, butylated polyvinylpyrrolidone (PVP), VP/ethyl methacrylate/methacrylic acid, VP/eicosene, VP/hexadecene, VP/triacontene, VP/styrene or VP/acrylic acid/lauryl methacrylate copolymer.

Not only for the staying power properties but also for the feel and consistency properties of the film, the PVP/hexadecene copolymer having an average molecular weight of from 7,000 to 7,500 or alternatively the PVP/eicosene copolymer having an average molecular weight of from 8,000 to 9,000 can be used.

The liposoluble or dispersible polymers in the composition of the invention can be also used in an amount of from 0.01% to 20% (as active material) relative to the total weight of the composition, such as, for example, from 1% to 10%, if they are present.

The basecoat composition according to the invention can be in the form of a tinted or non tinted dermatological composition or a care composition for keratin materials such as the skin, the lips and/or superficial body growths, in the form of an antisun composition or make-up-removing product in stick form. It can be used in particular as a care base for the skin, superficial body growths or the lips (lip balms, for protecting the lips against cold and/or sunlight and/or the wind, or care cream for the skin, the nails or the hair). As defined herein, a deodorant product is personal hygiene product and does not relate to care, make-up or treatment of keratin materials, including keratinous fibers.

The basecoat composition of the invention may also be in the form of a colored make-up product for the skin, in particular a foundation, optionally having care or treating properties, a blusher, a face powder, an eye shadow, a concealer product, an eyeliner, a make-up product for the body; a make-up product for the lips such as a lipstick, optionally having care or treating properties ; a make-up product for superficial body growths such as the nails or the eyelashes, in particular in the form of a mascara cake, or for the eyebrows and the hair, in particular in the form of a pencil.

Needless to say, the basecoat composition of the invention should be cosmetically or dermatologically acceptable, i.e., it should contain a non-toxic physiologically acceptable medium and should be able to be applied to the skin, superficial body growths or the lips of human beings. For the purposes of the invention, the expression "cosmetically acceptable" means a composition of pleasant appearance, odor, feel and/or taste.

The basecoat compositions of the present invention comprising at least one polyorganosiloxane containing polymer, preferably a silicone-polyamide copolymer, are applied topically to the desired area of the skin in an amount sufficient to make up the keratinous material, to cover or hide defects associated with keratinous material, skin imperfections or discolorations, or to enhance the appearance of keratinous material.

EXAMPLES

Lip gloss compositions in accordance with the present invention were prepared per the following formula:
All values are expressed in %w/w.
The following Examples 1-7 were prepared as follows:

- The oil of phase A was pre-heated to 100 °C for 10 minutes, with medium mixing, using a propeller mixer.
- Phase B (KRATON G1675 M) was added to phase A at 100°C.
- Phase B and phase A were mixed at high speed for 30 minutes until phase B was totally dissolved into phase A.
- Phase C (Regalite R1100) was then slowly added into phase (A+B) with medium mixing at 95 °C until the solution became homogeneous.
- The temperature was reduced to 90 °C and phase D containing oil mixtures was added to phase (A+B+C), and mixed at low speed.
- In a separate beaker Phase E ingredients were mixed, by hand, until the pigments were totally wet with oil to form a pigment mixture.
- The pigment mixture was then transferred to a three-roll mill and milled until the colors became homogeneous to form a milled pigment mixture.
- The milled pigment mixture was then transferred into a beaker containing phase (A+B+C+D) and mixed, at average speed, for approximately 5 minutes.
- Phase F was then slowly added into the beaker and mixed for 10 minutes at high speed.
- Mixing speed was then reduced and the resulting fluid transferred into individual packages at 90 °C.
- The samples contained in the packages were then cooled to room temperature.
- The samples exhibited desirable shine and wear properties.

EXAMPLE 1:

| PHASE | TRADE NAME | %w/w |
|---|---|---|
| A | FINSOLV TN [1] | 25.43 |
| B | KRATON G1657 M | 8.00 |
| C | REGALITE R1100 | 24.00 |
| D | PRISORINE 3631 [2] | 10.00 |
| | CRODAMOL STS | 24.26 |
| E | TITANIUM DIOXIDE | 0.15 |
| | IRON OXIDE | 0.13 |
| | D&C RED NO. 7 | 0.10 |
| | BLUE 1 LAKE | 0.01 |
| | BLACK IRON OXIDE | 0.04 |
| | FINSOLV TN [1] | 0.88 |
| | MICA | 3.00 |
| F | AMIHOPE LL [3] | 1.00 |
| | AEROSIL R972 | 3.00 |

(continued)

| PHASE | TRADE NAME | %w/w |
|---|---|---|
| | TOTAL | 100.00 |

[1]: C12-15 alkyl benzoate available from Finetex.
[2]: Pentaerythrityl tetraisostearate available from Uniqema.
[3]: Lauroyl Lysine powder available from Ajinomoto.

Rheology of example 1

- The elastic/storage modulus G', at a frequency of 0.01 rad/s, at a temperature of 25 °C, was 26.5 Pa, for the formulation of example 1.
- The creep viscosity, at a constant stress of 0.8 Pa, at a temperature of 25 °C, was $5.43 \times 10^3$ Pa.s, for the formulation of example 1.

EXAMPLE 2

| PHASE | TRADE NAME | %w/w |
|---|---|---|
| A | PURESYN [1] 2 | 25.76 |
| | ISOPROPYL PALMITATE | 10.00 |
| | FINSOLV TN [2] | 2.50 |
| B | KRATON G1657 M | 8.00 |
| C | REGALITE R1100 | 24.0 |
| D | PURESYN [1] 150 | 15.0 |
| | PURESYN 4E68 [3] | 7.00 |
| E | TITANIUM DIOXIDE | 0.15 |
| | IRON OXIDE | 0.31 |
| | D&C RED NO. 7 | 0.23 |
| | BLACK IRON OXIDE | 0.05 |
| | PURESYN 2 | 1.00 |
| F | MICA | 2.00 |
| | AMIHOPE LL [4] | 1.00 |
| | AEROSIL R972 | 3.00 |
| | TOTAL | 100.00 |

[1] : Hydrogenated polydecenes available from ExxonMobil..
[2] : C12-15 alkyl benzoate available from Finetex.
[3] : Pentaerythrityl tetraoleate available from ExxonMobil.
[4] : Lauroyl Lysine powder available from Ajinomoto.

Rheology of Example 2

- The elastic/storage modulus G', measured at a frequency of 0.01 rad/s and at a temperature of 25°C, was 14.4 Pa for the formulation of Example 2.
- The creep viscosity, at a constant stress of 0.8 Pa, at a temperature of 25 °C, was $1.55x10^3$ Pa.s, for formulation of Example 2.

EXAMPLE 3

| PHASE | TRADE NAME | %w/w |
|---|---|---|
| A | POLYSYNLANE LITE | 25.71 |
| | PURESYN [1] 6 | 12.00 |
| | ISOPROPYL PALMITATE | 8.00 |
| B | KRATON G1657 M | 9.00 |
| C | REGALITE R1100 | 18.00 |
| D | PURESYN [1] 150 | 6.00 |
| | DC 556 | 8.00 |
| | DC 555 | 4.00 |
| E | TITANIUM DIOXIDE | 0.15 |
| | IRON OXIDE | 0.31 |
| | D&C RED NO. 7 | 0.23 |
| | BENTONE VCG | 0.50 |
| | PROPYLENE CARBONATE | 0.05 |
| | BLACK IRON OXIDE | 0.05 |
| | POLYSYNLANE LITE | 2.00 |
| | POLYHYDROXYSTEARIC ACID | 1.00 |
| F | MICA | 2.00 |
| | AEROSIL R972 | 2.00 |
| | AMIHOPE LL [2] | 1.00 |
| | TOTAL | 100.00 |

[1]: Hydrogenated polydecenes available from ExxonMobil.
[2]: Lauroyl Lysine powder available from Ajinomoto.

Rheology of Example 3

The elastic/storage modulus G', at a frequency of 0.01 rad/s, at a temperature of 25°C, was 18.3 Pa, for Example 3.

The creep viscosity, at a constant stress of 0.8 Pa, 2 Pa, 5 Pa, and 7 Pa, at a temperature of 25 °C, was $2.32x10^4$ Pa.s, $4.32x10^2$ Pa.s, $7.98x10^1$ Pa.s and $5.60x10^1$ Pa.s, for Example 3.

EXAMPLE 4

| PHASE | TRADE NAME | %w/w |
|---|---|---|
| A | PURESYN [1] 2 | 25.26 |
| | ISOPROPYL PALMITATE | 8.00 |
| B | KRATON G1657 M | 8.00 |

(continued)

| PHASE | TRADE NAME | %w/w |
|---|---|---|
| C | REGALITE R1100 | 24.00 |
| D | PURESYN [1] 150 | 16.00 |
| | KF6038 [2] | 3.00 |
| E | TITANIUM DIOXIDE | 0.15 |
| | IRON OXIDE | 0.31 |
| | D&C RED NO. 7 | 0.23 |
| | BLACK IRON OXIDE | 0.05 |
| | PURESYN [1] 2 | 1.00 |
| F | MICA | 2.00 |
| | AEROSIL R972 | 3.00 |
| | AMIHOPE LL [3] | 1.00 |
| | TOTAL | 100.00 |

[1]: Hydrogenated polydecenes available from ExxonMobil.
[2]: Polyether/alkyl co-modified silicone available from Shin-Etsu.
[3]: Lauroyl Lysine powder available from Ajinomoto.

Rheology of example 4

The elastic/storage modulus G', at a frequency of 0.01 rad/s, at a temperature of 25°C, was 28.6 Pa for Examples 4.

The creep viscosity, at a constant stress of 0.8 Pa, at a temperature of 25 °C, was 1 5.34x$10^4$ Pa.s for Example 4.

EXAMPLES 5,6 et 7:

| PHASE | TRADE NAME | EXAMPLE 5 | EXAMPLE 6 | EXAMPLE 7 |
|---|---|---|---|---|
| A | PURESYN [1] 2 | 28.00 | 28.00 | 28.00 |
| | ISOPROPYL PALMITATE | 11.74 | 6.74 | 6.74 |
| B | KRATON G1657 M | 8.00 | 8.00 | 8.00 |
| C | REGALITE R1100 | 24.00 | 24.00 | 24.00 |
| D | PURESYN [1] 150 | 15.00 | 15.00 | 15.00 |
| | DC 555 | 0.00 | 0.00 | 5.00 |
| | DC 554 | 0.00 | 5.00 | 0.00 |
| E | TITANIUM DIOXIDE | 1.40 | 1.40 | 1.40 |
| | RED IRON OXIDE | 0.71 | 0.71 | 0.71 |
| | D&C RED N0. 7 | 0.96 | 0.96 | 0.96 |
| | FD&C YELLOW No 5 | 0.86 | 0.86 | 0.86 |
| | BLACK IRON OXIDE | 0.08 | 0.08 | 0.08 |
| | FINSOLV TN [2] | 3.25 | 3.25 | 3.25 |
| F | MICA | 3.00 | 3.00 | 3.00 |
| | AEROSIL R972 | 3.00 | 3.00 | 3.00 |
| | TOTAL | 100.00 | 100.00 | 100.00 |

[1] : Hydrogenated polydecenes available from ExxonMobil.
[2] : C12-15 alkyl benzoate available from Finetex.

Rheology of examples 5-7

The elastic/storage modulus G', at a frequency of 0.01 rad/s, at a temperature of 25 °C, was 35.3 Pa, 88.8 Pa, and 63.4 Pa, for Examples 5-7, respectively.

The creep viscosity, at a constant stress of 0.8 Pa, at a temperature of 25 °C, was 1.25X$10^4$ Pa.s, 5.54 X$10^4$ Pa.s, and 2.73 X$10^4$ Pa.s, for Examples 5 - 7, respectively.

EXAMPLE 8

An experiment was conducted to determine both the degree of shine and wear of shine of a cosmetic product in accordance with the present invention.

Basecoat Composition

| USA INCI name | Concentration |
|---|---|
| PROPYLENE CARBONATE | 0.8 |
| ISODODECANE | 59.7 |
| NYLON-611/DIMETHICONE COPOLYMER | 11 |
| LAUROYL LYSINE | 1.5 |
| Colorants | 7.5 |
| TRIMETHYLSILOXYSILICATE | 17 |
| DISTEARDIMONIUM HECTORITE | 2.5 |
| Total: | 100 |

Topcoat Composition

| USA INCI name | Concentration |
|---|---|
| HYDROGENATED STYRENE/METHYL STYRENE/INDENE COPOLYMER | 21 |
| ISOPROPYL PALMITATE | 9 |
| TRIMETHYL PENTAPHENYL TRISILOXANE | 5 |
| HYDROGENATED STYRENE/BUTADIENE COPOLYMER | 11 |
| HYDROGENATED POLYDECENE | 21 |
| PHENYL TRIMETHICONE | 9 |
| POLYISOBUTENE | 24 |
| Total: | 100 |

The experiment involved application of a basecoat composition, followed by a topcoat composition, onto a person's lips. Shine measurements were then taken at $T_0$ (initial); $T_1$ (after 1 hour); and $T_2$ (after 2 hours) to determine the wear of shine using the below-identified protocol.

Shine Measurement Protocol

In order to measure the shine of the above-mentioned cosmetic product, the intensity of the light used to perform the measurement was first determined and then its reflection off the surface of the lips was measured. This was done by having a first polarizer with vertical orientation in front of the light source, and a second polarizer with vertical orientation in front of a video camera. The video camera first recorded the surface reflection along with vertical light arising from any light passing through the gloss and into the lip.

The polarizer in front of the camera was then rotated by 90 degrees (at a video rate) in order to record the intensity of any vertical light transmitted below the surface. The horizontal intensity of sub-surface transmitted light was then measured. The second, horizontal measurement was a correction accounting for any sub-surface contributions to the desired, surface signal (the gloss). The second number was then subtracted from the first to yield the shine value.

Shine Kinetics Table

| | Shine measurement ($\pm$ 2) |
|---|---|
| $T_0$ | 181 |
| $T_1$ | 183 |
| $T_2$ | 172 |

As can be seen from the above data, the degree of shine possessed by the cosmetic product of the present invention when first applied is high. Surprisingly, however, after 1 hour the degree of shine remained fairly constant and even after 2 hours was extremely high, evidencing an exceptional wear of shine over time.

## Claims

1. A cosmetic composition comprising:

   (a) at least one block copolymer having at least one hard segment and at least one soft segment;
   (b) at least one tackifier component;
   (c) at least solvent capable of solubilizing the at least one hard segment; and
   (d) optionally, at least one colorant, and

   wherein the at least one hard segment has a $T_g$ value of 50°C or more, and the at least one soft segment has a $T_g$ value of 20°C or less,
   wherein (a) is a mixture of: (i) at least one di-block thermoplastic elastomer of an A-B type copolymer and (ii) at least one tri-block thermoplastic elastomer of an A-B-A type copolymer, wherein A corresponds to styrene and B corresponds to rubber,
   wherein the at least one tackifier is a hydrogenated styrene/methyl styrene/indene copolymer, and
   wherein the at least one solvent capable of solubilizing the at least one hard segment has a viscosity of from 0,001 to 0,2 Pa·s at room temperature and has a weight average molecular weight of from 100 to 500, with said solvent (c) being nonvolatile solvent, chosen among monoesters, diesters, triesters; mixed aliphatic and/or aromatic, polar oils; natural or synthetic esters of formula $R_1COOR_2$, wherein $R_1$ is a higher fatty residue comprising 7 to 19 carbon atoms, and $R_2$ is a branched hydrocarbon-based chain comprising 3 to 20 carbon atoms; synthetic esters of formula $R^3COR^4$, wherein $R^3$ is a $C_3$ to $C_{19}$ alkyl radical, and $R^4$ is a $C_3$ to $C_{20}$ alkyl radical; fatty alcohols comprising at least 12 carbon atoms; cyclic hydrocarbons; aromatic hydrocarbons; primary, secondary or tertiary amines and mixtures thereof.

2. The composition according to claim 1 wherein (a) has a styrene content of less than about 30% by weight, based on the weight of (a).

3. The composition according to anyone of the previous claims wherein (a) is present in the composition in an amount of from greater than 0% to 50% by weight, in particular in an amount of from greater than 0% to 10% by weight, based on the weight of the composition.

4. The composition according to anyone of the previous claims wherein the at least one tackifier component is present in the composition in an amount of from greater than 0% to 90% by weight, in particular from greater than 0% to 30% by weight, based on the weight of the composition.

5. The composition according to anyone of the previous claims wherein the tackifier has a solubility parameter corresponding to a number $\delta$ and the block copolymer has at least one segment with a solubility parameter corresponding to $\delta \pm 2$.

6. The composition according to anyone of the previous claims wherein the at least solvent capable of solubilizing the at least one hard segment has a viscosity of from about 0,001 to 0,2 Pa·s, in particular from 0,002 to 0,05 Pa·s. at room temperature.

7. The composition according to anyone of the previous claims wherein the at least solvent capable of solubilizing the at least one hard segment has a solubility parameter corresponding to a number $\delta'$ and the block copolymer has at least one hard segment with a solubility parameter from $\delta' \pm 2$ to $\delta' \pm 0.3$.

8. The composition according to anyone of the previous claims wherein the at least solvent capable of solubilizing the at least one hard segment has a weight average molecular weight of from 100 to 500.

9. The composition according to anyone of the previous claims further comprising at least one co-solvent having a weight average molecular weight of from 500 to 2500.

**10.** The composition according to anyone of the previous claims wherein the at least solvent capable of solubilizing the at least one hard segment is present in the composition in an amount of from greater than 0% to 85% by weight, in particular greater than 0% by weight to 60% by weight based on the weight of the composition.

**11.** The composition according to anyone of the previous claims wherein the at least solvent capable of solubilizing the at least one hard segment is chosen from linear or branched monoesters, diesters, triesters, and mixtures thereof.

**12.** The composition according to anyone of the previous claims wherein the at least solvent capable of solubilizing the at least one hard segment is an ester chosen from $C_{12-15}$ alkyl benzoate, isopropyl myristate, isopropyl palmilate, isononyl isononanoate, cetyl ethylhexanoate, neopentyl glycol diethylhexanoate, diisopropyl sebacate.

**13.** The composition according to anyone of the previous claims wherein it further comprises at least one solvent capable of solubilizing the at least one soft segment.

**14.** The composition according to the previous claim wherein the at least one solvent capable of solubilizing the soft segment has a viscosity of from 0,001 to 0,5 Pa·s, in particular from 0,002 to 0,02 Pa·s at room temperature.

**15.** The composition of claim 13 or 14wherein at least one solvent capable of solubilizing the soft segment has a solubility parameter corresponding to a number $\delta'$ and the block copolymer has at least one soft segment with a solubility parameter from $\delta' \pm 2$ to $\delta' \pm 0.3$.

**16.** The composition according to anyone of the claims. 13 to 15 wherein the at least one solvent capable of solubilizing the soft segment has a weight average molecular weight of from 150 to 450.

**17.** The composition according to anyone of the previous claims wherein it comprises at least one co-solvent having a weight average molecular weight of from 500 to 2000 optionally in an amount of from greater than 0% by weight to about 60% by weight, based on the weight of the composition.

**18.** The composition according to anyone of the claims 13 to 17 wherein the at least one solvent capable of solubilizing the soft segment is chosen from polybutene, hydrogenated polybutene, polyisobutene, hydrogenated polyisobutene, isoeicosane, polydecene and hydrogenated polydecene.

**19.** The composition according to anyone of the previous claims wherein the colorant is present in an amount effective to impart color when applied onto lips.

**20.** The composition according to anyone of the previous claims wherein it further comprises at least one modified silicone optionally in an amount of from greater than 0% by weight to 30% by weight, based on the weight of the composition.

**21.** The composition of the previous claim wherein the at least one modified silicone is chosen from alkyl modified silicones, ester modified silicones, fluorosilicones, and mixtures thereof.

**22.** The composition according to anyone of the previous claims wherein it further comprises at least one gelling agent optionally in an amount of from 0.1% to 20% by weight,in particular from 0.1% to 10% by weight based on the weight of the composition.

**23.** The composition of claim 22, wherein the at least one gelling agent is a fumed silica, a hydrophobic silica and/or a modified clay in particular selected from hectorites, bentonites, quaternium-18 bentonite, stearalkonium, bentonites and mixtures thereof.

**24.** The composition according to anyone of the previous claims wherein it further comprises at least one shine enhancing agent optionally in an amount of from greater than 0% to 30% by weight, in particular from greater than 0% to 20% by weight based on the weight of the composition.

**25.** The composition of the previous claim wherein the at least one shine enhancing agent is a phenylated silicone optionally having an average number molecular weight of less than 10,000, in particular less than 2,000.

**26.** The composition according to anyone of the previous claims wherein the composition has an elastic/storage modulus

G', at a frequency ω of 0.01 rad/s, ranging from 0.01 Pa to 500 Pa at 25 °C.

**27.** The composition according to anyone of the previous claims wherein the composition has a creep viscosity ($\eta_{creep}$) ranging from 2 Pa.s to 150,000 Pa.s at 25 °C.

**28.** A cosmetic process for treating lips comprising contacting the lips with the composition according to anyone of the previous claims.

**29.** A cosmetic method of making-up a keratinous substrate comprising:

(a) providing a keratinous substrate;
(b) applying a basecoat composition onto the keratinous substrate, the basecoat composition comprising:

(i) at least one polyorganosiloxane containing polymer chosen among

(1) the (co)polymers comprising at least one organosiloxane unit and at least two other groups capable of forming hydrogen interactions chosen from an ester group, a sulfonamide group, a carbamate group, a thiocarbamate group, a urea group, a thio-urea group, an oxamido group, a guanidino group, a biguanidino group, an amide group, and mixtures thereof;
(2) the silicone polyamide copolymers and their mixtures

(ii) at least one silicone film former;
(iii)at least one volatile oil; and
(iv) at least one colorant; and

(c) applying a topcoat composition over top of the basecoat composition, the topcoat composition being as defined in the previous claims 1 to 27.

**30.** The method of claim 29 wherein (b)(i) is present in an amount of from about 0.5 to about 30% by weight, in particular from about 1 to about 20% by weight based on the weight of the basecoat composition.

**31.** The method according of claim 29 or 30, wherein (b)(ii) is a trimethylsiloxysilicate.

**32.** The method according to anyone of the claims 29 to 31 wherein (b)(ii) is present in an amount of from 0.1 to 30% by weight, in particular from 0.5 to about 20% by weight based on the weight of the basecoat composition.

**33.** The method according to anyone of previous claims 29 to 32 wherein the keratinous substrate is lips.

**34.** The method according to anyone of previous claims 29 to 32 wherein the keratinous substrate is hair.

**35.** The method according to anyone of previous claims 29 to 34 wherein the topcoat composition further comprises at least one short chain ester optionally in an amount of from greater than 0% to 20% by weight, in particular from greater than 0% to 15% by weight, and more particularly from greater than 0% to 10% by weight, based on the weight of the topcoat composition.

**36.** The method according to anyone of previous claims 30 to 35 wherein (b)(i) is chosen from the silicone-polyester copolymers, silicone-polycarbamate copolymers, silicone-sulfonamide copolymers, silicone-urea copolymers, silicone-thio-urea copolymers, silicone-oxamido copolymers, silicone-guanidino copolymers, silicone-biguanidino copolymers and mixtures thereof.

## Patentansprüche

**1.** Kosmetische Zusammensetzung, umfassend:

(a) mindestens ein Blockcopolymer mit mindestens einem harten Segment und mindestens einem weichen Segment;
(b) mindestens eine klebrigmachende Komponente;

(c) mindestens Lösungsmittel, welches zum Solubilisieren des mindestens einen harten Segments in der Lage ist; und

(d) gegebenenfalls mindestens ein farbgebendes Mittel, und

wobei das mindestens eine harte Segment einen $T_g$-Wert von 50°C oder höher aufweist und das mindestens eine weiche Segment einen $T_g$-Wert von 20°C oder niedriger aufweist,
wobei (a) ein Gemisch ist von: (i) mindestens einem thermoplastischen Diblockelastomer eines Copolymers vom A-B-Typ und (ii) mindestens einem thermoplastischen Triblockelastomer eines Copolymers vom A-B-A-Typ, wobei A Styrol entspricht und B Kautschuk entspricht,
wobei das mindestens eine klebrigmachende Mittel ein hydriertes Styrol/Methylstyrol/Inden-Copolymer ist, und wobei das mindestens eine Lösungsmittel, welches zum Solubilisieren des mindestens einen harten Segments in der Lage ist, eine Viskosität von 0,001 bis 0,2 Pa·s bei Raumtemperatur aufweist und ein gewichtsmittleres Molekulargewicht von 100 bis 500 aufweist, wobei das Lösungsmittel (c) nicht-flüchtiges Lösungsmittel ist, welches ausgewählt ist aus Monoestern, Diestern, Triestern; aliphatischen und/oder aromatischen, polaren Ölgemischen; natürlichen oder synthetischen Estern der Formel $R_1COOR_2$, wobei $R_1$ ein höherer fetter Rest, umfassend 7 bis 19 Kohlenstoffatome, ist und $R_2$ eine verzweigte Kette auf Kohlenwasserstoffbasis, umfassend 3 bis 20 Kohlenstoffatome, ist; synthetischen Estern der Formel $R^3COR^4$, wobei $R^3$ ein $C_3$- bis $C_{19}$-Alkylrest ist und $R^4$ ein $C_3$- bis $C_{20}$-Alkylrest ist; fetten Alkoholen, umfassend mindestens 12 Kohlenstoffatome; cyclischen Kohlenwasserstoffen; aromatischen Kohlenwasserstoffen; primären, sekundären oder tertiären Aminen und Gemischen davon.

2. Zusammensetzung gemäß Anspruch 1, wobei (a) einen Styrolgehalt von niedriger als etwa 30 Gew.-%, bezogen auf das Gewicht von (a), aufweist.

3. Zusammensetzung gemäß einem der vorhergehenden Ansprüche, wobei (a) in der Zusammensetzung in einer Menge von höher als 0 Gew.-% bis 50 Gew.-%, insbesondere in einer Menge von höher als 0 Gew.-% bis 10 Gew.-%, bezogen auf das Gewicht der Zusammensetzung, vorhanden ist.

4. Zusammensetzung gemäß einem der vorhergehenden Ansprüche, wobei die mindestens eine klebrigmachende Komponente in der Zusammensetzung in einer Menge von höher als 0 Gew.-% bis 90 Gew.-%, insbesondere von höher als 0 Gew.-% bis 30 Gew.-%, bezogen auf das Gewicht der Zusammensetzung, vorhanden ist.

5. Zusammensetzung gemäß einem der vorhergehenden Ansprüche, wobei das klebrigmachende Mittel einen Löslichkeitsparameter entsprechend einer Zahl $\delta$ aufweist und das Blockcopolymer mindestens ein Segment mit einem Löslichkeitsparameter entsprechend $\delta \pm 2$ aufweist.

6. Zusammensetzung gemäß einem der vorhergehenden Ansprüche, wobei das mindestens Lösungsmittel, welches zum Solubilisieren des mindestens einen harten Segments in der Lage ist, eine Viskosität von etwa 0,001 bis 0,2 Pa·s, insbesondere von 0,002 bis 0,05 Pa·s bei Raumtemperatur aufweist.

7. Zusammensetzung gemäß einem der vorhergehenden Ansprüche, wobei das mindestens Lösungsmittel, welches zum Solubilisieren des mindestens einen harten Segments in der Lage ist, einen Löslichkeitsparameter entsprechend einer Zahl $\delta'$ aufweist und das Blockcopolymer mindestens ein hartes Segment mit einem Solubilisierungsparameter von $\delta' \pm 2$ bis $\delta' \pm 0{,}3$ aufweist.

8. Zusammensetzung gemäß einem der vorhergehenden Ansprüche, wobei das mindestens Lösungsmittel, welches zum Solubilisieren des mindestens einen harten Segments in der Lage ist, ein gewichtsmittleres Molekulargewicht von 100 bis 500 aufweist.

9. Zusammensetzung gemäß einem der vorhergehenden Ansprüche, ferner umfassend mindestens ein Co-Lösungsmittel mit einem gewichtsmittleren Molekulargewicht von 500 bis 2500.

10. Zusammensetzung gemäß einem der vorhergehenden Ansprüche, wobei das mindestens Lösungsmittel, welches zum Solubilisieren des mindestens einen harten Segments in der Lage ist, in der Zusammensetzung in einer Menge von höher als 0 Gew.-% bis 85 Gew.-%, insbesondere höher als 0 Gew.-% bis 60 Gew.-%, bezogen auf das Gewicht der Zusammensetzung, vorhanden ist.

11. Zusammensetzung gemäß einem der vorhergehenden Ansprüche, wobei das mindestens Lösungsmittel, welches zum Solubilisieren des mindestens einen harten Segments in der Lage ist, aus linearen oder verzweigten Monoestern, Diestern, Triestern und Gemischen davon ausgewählt ist.

**12.** Zusammensetzung gemäß einem der vorhergehenden Ansprüche, wobei das mindestens Lösungsmittel, welches zum Solubilisieren des mindestens einen harten Segments in der Lage ist, ein Ester, ausgewählt aus $C_{12-15}$-Alkylbenzoat, Isopropylmyristat, I-sopropylpaimitat, Isononylisononanoat, Cetylethylhexanoat, Neopentylglycoldiethylhexanoat, Diisopropylsebacat, ist.

**13.** Zusammensetzung gemäß einem der vorhergehenden Ansprüche, wobei sie ferner mindestens ein Lösungsmittel, welches zum Solubilisieren des mindestens einen weichen Segments in der Lage ist, umfasst.

**14.** Zusammensetzung gemäß dem vorhergehenden Anspruch, wobei das mindestens eine Lösungsmittel, welches zum Solubilisieren des weichen Segments in der Lage ist, eine Viskosität von 0,001 bis 0,05 Pa·s, insbesondere von 0,002 bis 0,02 Pa·s bei Raumtemperatur aufweist.

**15.** Zusammensetzung nach Anspruch 13 oder 14, wobei mindestens ein Lösungsmittel, welches zum Solubilisieren des weichen Segments in der Lage ist, einen Löslichkeitsparameter entsprechend einer Zahl δ' aufweist und das Blockcopolymer mindestens ein weiches Segment mit einem Löslichkeitsparameter von δ' ± 2 bis δ' ± 0,3 aufweist.

**16.** Zusammensetzung gemäß einem der Ansprüche 13 bis 15, wobei das mindestens eine Lösungsmittel, welches zum Solubilisieren des weichen Segments in der Lage ist, ein gewichtsmittleres Molekulargewicht von 150 bis 450 aufweist.

**17.** Zusammensetzung gemäß einem der vorhergehenden Ansprüche, wobei sie mindestens ein Co-Lösungsmittel mit einem gewichtsmittleren Molekulargewicht von 500 bis 2000, gegebenenfalls in einer Menge von höher als 0 Gew.-% bis etwa 60 Gew.-%, bezogen auf das Gewicht der Zusammensetzung, umfasst.

**18.** Zusammensetzung gemäß einem der Ansprüche 13 bis 17, wobei das mindestens eine Lösungsmittel, welches zum Solubilisieren des weichen Segments in der Lage ist, aus Polybuten, hydriertem Polybuten, Polyisobuten, hydriertem Polyisobuten, Isoeicosan, Polydecen und hydriertem Polydecen ausgewählt ist.

**19.** Zusammensetzung gemäß einem der vorhergehenden Ansprüche, wobei das farbgebende Mittel in einer Menge vorhanden ist, welche zum Verleihen von Farbe wirksam ist, wenn sie auf Lippen aufgebracht wird.

**20.** Zusammensetzung gemäß einem der vorhergehenden Ansprüche, wobei sie ferner mindestens ein modifiziertes Silikon, gegebenenfalls in einer Menge von höher als 0 Gew.-% bis 30 Gew.-%, bezogen auf das Gewicht der Zusammensetzung, umfasst.

**21.** Zusammensetzung nach dem vorhergehenden Anspruch, wobei das mindestens eine Silikon aus Alkyl-modifizierten Silikonen, Ester-modifizierten Silikonen, Fluorsiliconen und Gemischen davon ausgewählt ist.

**22.** Zusammensetzung gemäß einem der vorhergehenden Ansprüche, wobei sie ferner mindestens ein Geliermittel, gegebenenfalls in einer Menge von 0,1 Gew.-% bis 20 Gew.-%, insbesondere von 0,1 Gew.-% bis 10 Gew.-%, bezogen auf das Gewicht der Zusammensetzung, umfasst.

**23.** Zusammensetzung nach Anspruch 22, wobei das mindestens eine Geliermittel ein Quarzstaub, ein hydrophobes Siliciumdioxid und/oder ein modifizierter Ton, insbesondere ausgewählt aus Hektoriten, Bentoniten, Quaternium-18-Bentonit, Stearalkonium, Bentoniten und Gemischen davon, ist.

**24.** Zusammensetzung gemäß einem der vorhergehenden Ansprüche, wobei sie ferner mindestens ein glanzsteigerndes Mittel, gegebenenfalls in einer Menge von höher als 0 Gew.-% bis 30 Gew.-%, insbesondere von höher als 0 Gew.-% bis 20 Gew.-%, bezogen auf das Gewicht der Zusammensetzung, umfasst.

**25.** Zusammensetzung nach dem vorhergehenden Anspruch, wobei das mindestens eine glanzsteigerende Mittel ein phenyliertes Silikon, gegebenenfalls mit einem zahlenmittleren Molekulargewicht von niedriger als 10.000, insbesondere niedriger als 2.000, ist.

**26.** Zusammensetzung gemäß einem der vorhergehenden Ansprüche, wobei die Zusammensetzung ein Elastizitäts-/Lagermodul G' bei einer Frequenz ω von 0,01 rad/s im Bereich von 0,01 Pa bis 500 Pa bei 25°C aufweist.

**27.** Zusammensetzung gemäß einem der vorhergehenden Ansprüche, wobei die Zusammensetzung eine Kriechviskosität ($\eta_{Kriech}$) im Bereich von 2 Pa·s bis 150.000 Pa·s bei 25°C aufweist.

**28.** Kosmetisches Verfahren zum Behandeln von Lippen, umfassend das Inkontaktbringen der Lippen mit der Zusammensetzung gemäß einem der vorhergehenden Ansprüche.

**29.** Kosmetisches Verfahren zum Aufbringen von Make-up auf ein keratinöses Substrat, umfassend:

(a) das Bereitstellen eines keratinösen Substrats;
(b) das Aufbringen einer Grundschichtzusammensetzung auf das keratinöse Substrat, wobei die Grundschichtzusammensetzung umfasst:

(i) mindestens ein Polyorganosiloxan-enthaltendes Polymer, welches ausgewählt ist aus

(1) den (Co)polymeren, umfassend mindestens eine Organosiloxaneinheit und mindestens zwei andere Reste, welche zum Bilden von Wasserstoffwechselwirkungen in der Lage sind, ausgewählt aus einer Estergruppe, einer Sulfonamidgruppe, einer Carbamatgruppe, einer Thiocarbamatgruppe, einer Harnstoffgruppe, einer Thioharnstoffgruppe, einer Oxamidogruppe, einer Guanidinogruppe, einer Biguanidinogruppe, einer Amidgruppe, und Gemischen davon;
(2) den Silicon-Polyamid-Copolymeren und ihren Gemischen

(ii) mindestens ein Siliconfilm-bildendes Mittel;
(iii) mindestens ein flüchtiges Öl; und
(iv) mindestens ein farbgebendes Mittel; und

(c) das Aufbringen einer Deckschichtzusammensetzung über der Grundschichtzusammensetzung, wobei die Deckschichtzusammensetzung so wie in den vorhergehenden Ansprüchen 1 bis 27 definiert ist.

**30.** Verfahren nach Anspruch 29, wobei (b) (i) in einer Menge von etwa 0,5 bis etwa 30 Gew.-%, insbesondere von etwa 1 bis etwa 20 Gew.-%, bezogen auf das Gewicht der Grundschichtzusammensetzung, vorhanden ist.

**31.** Verfahren gemäß Anspruch 29 oder 30, wobei (b) (ii) ein Trimethylsiloxysilicat ist.

**32.** Verfahren gemäß einem der Ansprüche 29 bis 31, wobei (b) (ii) in einer Menge von 0,1 bis 30 Gew.-%, insbesondere von 0,5 bis etwa 20 Gew.-%, bezogen auf das Gewicht der Grundschichtzusammensetzung, vorhanden ist.

**33.** Verfahren gemäß einem der vorhergehenden Ansprüche 29 bis 32, wobei das keratinöse Substrat Lippen sind.

**34.** Verfahren gemäß einem der vorhergehenden Ansprüche 29 bis 32, wobei das keratinöse Substrat Haar ist.

**35.** Verfahren gemäß einem der vorhergehenden Ansprüche 29 bis 34, wobei die Deckschichtzusammensetzung ferner mindestens einen kurzkettigen Ester, gegebenenfalls in einer Menge von höher als 0 Gew.-% bis 20 Gew.-%, insbesondere von höher als 0 Gew.-% bis 15 Gew.-% und stärker besonders von höher als 0 Gew.-% bis 10 Gew.-%, bezogen auf das Gewicht der Deckschichtzusammensetzung, umfasst.

**36.** Verfahren gemäß einem der vorhergehenden Ansprüche 30 bis 35, wobei (b) (i) von den Silikon-Polyester-Copolymeren, Silikon-Polycarbamat-Copolymeren, Silikon-Sulfonamid-Copolymeren, Silikon-Harnstoff-Copolymeren, Silikon-Thioharnstoff-Copolymeren, Silikon-Oxamido-Copolymeren, Silikon-Guanidino-Copolymeren, Silikon-Biguanidino-Copolymeren und Gemischen davon ausgewählt ist.

## Revendications

**1.** Composition cosmétique comprenant :

(a) au moins un copolymère bloc ayant au moins un segment rigide et au moins un segment souple ;
(b) au moins un composant d'adhésivité ;
(c) au moins un solvant capable de solubiliser le au moins un segment rigide ; et

(d) éventuellement, au moins un colorant, et

dans laquelle le au moins un segment rigide a une valeur $T_g$ de 50°C ou plus, et le au moins un segment souple a une valeur $T_g$ de 20°C ou moins,
dans laquelle (a) est un mélange de : (i) au moins un élastomère thermoplastique di-bloc d'un copolymère de type A-B et (ii) au moins un élastomère thermoplastique tri-bloc d'un copolymère de type A-B-A, dans lequel A correspond au styrène et B correspond au caoutchouc,
dans laquelle le au moins un agent d'adhésivité est un copolymère styrène hydrogéné/méthylstyrène/indène, et
dans laquelle le au moins un solvant capable de solubiliser le au moins un segment rigide a une viscosité de 0,001 à 0,2 Pa.S à température ambiante et a un poids moléculaire moyen de 100 à 500, ledit solvant (c) étant un solvant non volatile, choisi parmi des monoesters, diesters, triesters ; des huiles aliphatiques et/ou aromatiques, polaires en mélange ; des esters naturels ou synthétiques de formule $R_1COOR_2$, dans laquelle $R_1$ est un résidu d'acide gras supérieur comprenant 7 à 19 atomes de carbone, et $R_2$ est une chaîne ramifiée à base d'hydrocarbure comprenant 3 à 20 atomes de carbone ; des esters synthétiques de formule $R_3COR_4$, dans laquelle $R_3$ est un radical alkyle en $C_3$-$C_{19}$, et $R_4$ est un radical alkyle en $C_3$-$C_{20}$ ; des alcools gras comprenant au moins 12 atomes de carbone ; des hydrocarbures cycliques ; des hydrocarbures aromatiques ; des amines primaires, secondaires ou tertiaires et des mélanges de ceux-ci.

**2.** Composition selon la revendication 1 dans laquelle (a) a un contenu en styrène de moins d'environ 30 % en poids, rapporté au poids de (a).

**3.** Composition selon l'une quelconque des revendications précédentes dans laquelle le au moins un composant d'adhésivité est présent dans la composition en une quantité de plus de 0 % à 50 % en poids, en particulier de plus de 0 % à 10 % en poids, rapportéee au poids de la composition.

**4.** Composition selon l'une quelconque des revendications précédentes dans laquelle (a) est présent dans la composition en une quantité de plus de 0 % à 90% en poids, en particulier en une quantité de plus de 0 % à 30% en poids, rapporté au poids de la composition.

**5.** Composition selon l'une quelconque des revendications précédentes dans laquelle l'agent d'adhésivité a un paramètre de solubilité correspondant à un nombre δ et le copolymère bloc a au moins un segment ayant un paramètre de solubilité correspondant à $\delta \pm 2$.

**6.** Composition selon l'une quelconque des revendications précédentes dans laquelle le au moins un solvant capable de solubiliser le au moins un segment rigide a une viscosité d'environ 0,001 à 0,2 Pa.S en particulier de 0,002 à 0,05 Pa.S à température ambiante.

**7.** Composition selon l'une quelconque des revendications précédente dans laquelle le au moins un solvant capable de solubiliser le au moins un segment rigide a un paramètre de solubilité correspondant à un nombre δ' et le copolymère bloc a au moins un segment rigide ayant un paramètre de $\delta' \pm 2$ à $\delta' \pm 0{,}3$.

**8.** Composition selon l'une quelconque des revendications précédentes dans laquelle le au moins un solvant capable de solubiliser le au moins un segment rigide a un poids moléculaire moyen de 100 à 500.

**9.** Composition selon l'une quelconque des revendications précédentes comprenant en outre au moins un co-solvant ayant un poids moléculaire moyen de 500 à 2500.

**10.** Composition selon l'une quelconque des revendications précédentes dans laquelle le au moins un solvant capable de solubiliser le au moins un segment rigide est présent dans la composition de plus de 0 % à 85 % en poids, en particulier de plus de 0 % en poids à 60 % en poids, rapportée au poids de la composition.

**11.** Composition selon l'une quelconque des revendications précédentes dans laquelle le au moins un solvant capable de solubiliser le au moins un segment rigide est choisi parmi des mononesters, diesters, triesters linéaires ou ramifiés et des mélanges de ceux-ci.

**12.** Composition selon l'une quelconque des revendications précédentes dans laquelle le au moins un solvant capable de solubiliser le au moins un segment rigide est un ester choisi parmi un alkylbenzoate en $C_{12}$-$C_{15}$, isopropylmyristate, isopropylpalmilate, isononyl-isononanoate, cétyléthyhexanoate, néopentylglycol, diéthylhexanoate, diisopropylsébacate.

**13.** Composition selon l'une quelconque des revendications précédentes qui comprend en outre au moins un solvant capable de solubiliser le au moins un segment souple.

**14.** Composition selon la revendications précédente dans laquelle le au moins un solvant capable de solubiliser le segment souple a une viscosité de 0,001 à 0,05 Pa.S, en particulier de 0,002 à 0,02 Pa.S à température ambiante.

**15.** Composition de la revendication 13 ou 14 dans laquelle le au moins un solvant capable de solubiliser le segment souple a un paramètre de solubilité correspondant a un nombre δ' et le polymère bloc a au moins un segment souple ayant un paramètre de solubilité de δ' ± 2 à δ' ± 0,3 .

**16.** Composition selon l'une quelconque des revendications 13 à 15 dans laquelle le au moins un solvant capable de solubiliser le au moins un segment souple a un poids moléculaire moyen de 150 à 450.

**17.** Composition selon l'une quelconque des revendications précédentes qui comprend au moins un co-solvant ayant un poids moléculaire moyen de 500 à 2000 éventuellement en une quantité de plus de 0 % en poids à environ 60 % en poids, rapportée au poids de la composition.

**18.** Composition selon l'une quelconque des revendications 13 à 17 dans laquelle le au moins un solvant capable de solubiliser le segment souple est choisi parmi le polybutène, polybutène hydrogéné, polyisobutène, polyisobutène hydrogéné/isoéicosane, polydécène et polydécène hydrogéné.

**19.** Composition selon l'une quelconque des revendications précédentes dans laquelle le colorant est présent en une quantité efficace pour donner une couleur lorsqu'il est appliqué sur les lèvres.

**20.** Composition selon l'une quelconque des revendications précédentes qui comprend en outre au moins un silicone modifié éventuellement en une quantité de plus de 0 % en poids à 30 % en poids, rapportée au poids de la composition.

**21.** Composition de la revendication précédente dans laquelle le au moins un silicone modifié est choisi parmi des silicones modifiés par un alkyle, des silicones modifiés par un ester, des fluorosilicones et des mélanges de ceux-ci.

**22.** Composition selon l'une quelconque des revendications précédentes qui comprend en outre au moins un agent gélifiant éventuellement en une quantité de plus de 0,1% à 20 % en poids, en particulier de 0,1% à 10 % en poids, rapportée au poids de la composition.

**23.** Composition de la revendication 22, dans laquelle le au moins un agent gélifiant est une silice fumée, une silice hydrophobe et/ou une argile modifiée en particulier choisie parmi des hectorites, bentonites, quaternium-18 bentonite, stéaralkonium, bentonites et des mélanges de ceux-ci.

**24.** Composition selon l'une quelconque des revendications précédentes qui comprend en outre au moins un agent rehausseur de brillance éventuellement en une quantité de plus de 0% à 30 % en poids, en particulier de 0% à 20 % en poids, rapportée au poids de la composition.

**25.** Composition de la revendication précédente dans laquelle le au moins un rehausseur de brillance est un silicone phénylé ayant un poids moléculaire moyen de moins de 10 000, en particulier moins de 2 000.

**26.** Composition selon l'une quelconque des revendications précédentes dans laquelle la composition a un module élastique/de stockage G', à une fréquence ω de 0,01 rad/s, allant de 0,01 Pa à 500 Pa à 25°C

**27.** Composition selon l'une quelconque des revendications précédentes dans laquelle la composition a une viscosité de fluage ($\eta_{fluage}$) allant de 2 Pa.S à 150 000 Pa.S à 25°C.

**28.** Procédé cosmétique pour le traitement des lèvres comprenant la mise en contact des lèvres avec la composition selon l'une quelconque des revendications précédentes.

**29.** Méthode cosmétique de maquillage d'un substrat kératineux comprenant :

(a) la fourniture d'un substrat kératineux ;
(b) l'application d'une composition de couche de base sur le substrat kératineux, la composition de couche de base comprenant :

(i) au moins un polyorgnaosiloxane contenant un polymère choisi parmi

(1) les (co)-polymères comprenant au moins une unité organosiloxane et au moins deux autres groupements capables de former des interactions hydrogène choisis parmi un groupement ester, un groupement sulfonamide, un groupement carbamate, un groupement thiocarbamate, un groupement urée, un groupement thio-urée, un groupement oxamido, un groupement guanidino, un groupement biguanidino, un groupement amide et des mélanges de ceux-ci ;
(2) les copolymères de polyamide de silicone et leurs mélanges

(ii)au moins un agent filmogène à base de silicone ;
(iii)au moins une huile volatile ; et
(iv) au moins un colorant, et

(c) l'application d'une composition de couche de finition par dessus la composition de couche de base, la composition de la couche de finition étant définie dans les revendications précédentes 1 à 27.

**30.** Méthode de la revendication 29 dans laquelle (b) (i) est présent en une quantité d'environ 0,5 à environ 30 % en poids, en particulier d'environ 1 à environ 20 % en poids rapportée au poids de la composition de la couche de base.

**31.** Méthode selon la revendication 29 ou 30, dans laquelle (b) (ii) est un triméthylsiloxysilicate.

**32.** Méthode selon l'une quelconque des revendications 29 à 31 dans laquelle (b) (ii) est présent en une quantité de 0,1 à 30 % en poids, en particulier de 0,5 à environ 20 % en poids, rapportée au poids de la composition de couche de base.

**33.** Méthode selon l'une quelconque des revendications précédentes 29 à 32 dans laquelle le substrat kératineux est les lèvres.

**34.** Méthode selon l'une quelconque des revendications précédentes 29 à 32 dans laquelle le substrat kératineux est les cheveux.

**35.** Méthode selon l'une quelconque des revendications précédentes 29 à 34 dans laquelle la composition de la couche de finition comprend en outre au moins une chaîne ester courte éventuellement en une quantité de plus de 0 % à 20 % en poids, en particulier de plus de 0 % à 15 % en poids, et plus particulièrement de plus de 0 % à 10 % en poids, rapportée au poids de la composition de couche de finition.

**36.** Méthode selon l'une quelconque des revendications précédentes 30 à 35 dans laquelle (b) (i) est choisi parmi les copolymères silicone-polyester, copolymères silicone-polycarbamate, copolymères silicone-sulfonamide, copolymères silicone-urée, copolymères silicone-thio-urée, copolymères silicone-oxamido, copolymères silicone-guanidino, copolymères silicone-biguanidino et des mélanges de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5294438 A
- US 6403070 B
- US 6656458 B
- US 5874069 A
- US 5919441 A
- US 6051216 A
- US 5981680 A
- US 6503632 B
- US 6569955 B
- US 6338839 B
- WO 03042221 A
- US 5246694 A
- US 5061481 A
- US 5219560 A
- US 5262087 A
- US 6045782 A
- US 5334737 A
- US 4725658 A
- US 5209924 A
- US 4972037 A
- WO 0132737 A
- WO 0132727 A1
- US 4693935 A
- US 4981903 A
- US 4981902 A
- US 5468477 A

### Non-patent literature cited in the description


- **ERIC A. GRULKE.** Solubility Parameter Values. *Polymer Handbook,* 519-559
- **C. M. HANSEN.** The three-dimensional solubility parameters. *J. Paint Technol.,* 1967, vol. 39, 105